# EUROPEAN PATENT APPLICATION

(11) **EP 4 206 799 A1**
(43) Date of publication of application: **05.07.2023**
(21) Application number: 21877629.2
(22) Date of filing: 05.10.2021
(51) Int. Cl.: G02C 7/10, G02B 5/23

(54) **OPTICAL ARTICLE, EYEGLASSES AND PHOTOCHROMIC COMPOUND**

(30) Priority: 05.10.2020 US 202063087450 P
(71) Applicant: Hoya Lens Thailand Ltd., Pathumthani 12130 (TH)
(72) Inventor: KAWAKAMI Hironori, Tokyo 160-8347 (JP); MATSUE Aoi, Tokyo 160-8347 (JP); KOBAYASHI Kei, Tokyo 160-8347 (JP)
(74) Representative: Hoffmann Eitle
(86) International application number: PCT/JP2021/036860
(87) International publication number: WO 2022/075330

(57) **Abstract**

Provided is an optical article including one or more photochromic compounds selected from the group consisting of a compound represented by general formula 1 and a compound represented by general formula 2. In the general formula 1, R¹ to R⁸, and B¹ and B² each independently represent a hydrogen atom or a substituent, and at least either of B¹ and B² represents a monovalent group represented by general formula 3; in the general formula 2, R⁹ to R¹⁶, and B³ and B⁴ each independently represent a hydrogen atom or a substituent, and at least either of B³ and B⁴ represents a monovalent group represented by general formula 3; and in the general formula 3, Ar¹ represents an aryl group or a heteroaryl group, R¹⁸ represents a hydrogen atom or a substituent, Ar¹ and R¹⁸ may be coupled to form a ring structure via a single bond or a divalent linking group, and * represents a bonding position to an adjacent carbon atom).

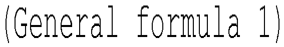

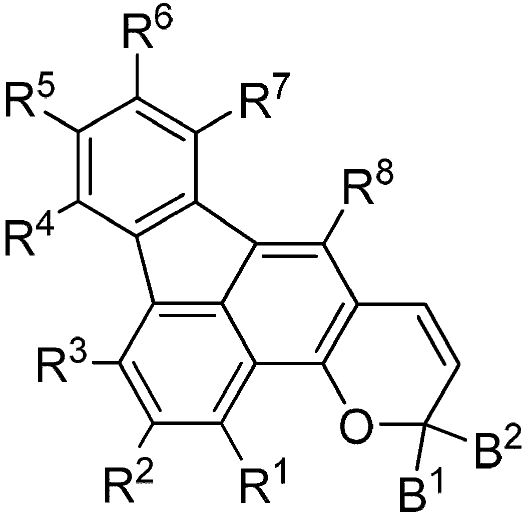

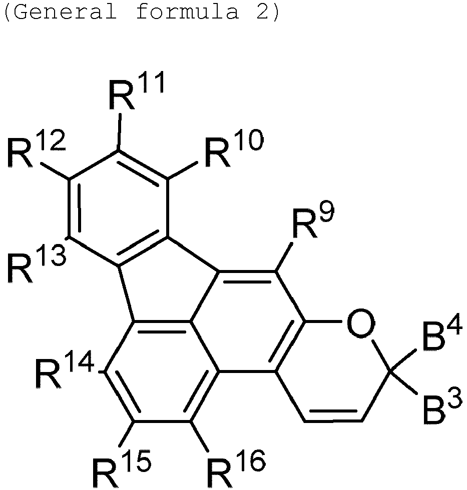

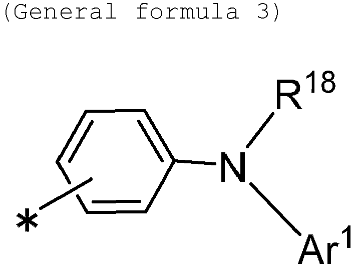

## Description

### [Technical Field]

The present invention relates to an optical article, spectacles, and a photochromic compound.

### [Background Art]

A photochromic compound is a compound having the property of developing a color (coloring) under irradiation with light within a wavelength range having photoresponsivity and fading under the unirradiated state (photochromic properties). For example, PTL 1 discloses a naphthopyran-based compound as a photochromic compound.

### [Citation List]

### [Patent Literature]

[PTL 1] WO 2000/15631

### [Summary of Invention]

### [Technical Problem]

As a method for imparting photochromic properties to an optical article, such as a spectacle lens, a method for incorporating a photochromic compound into a substrate, and a method for forming a layer containing a photochromic compound (a photochromic layer) may be mentioned. As a property desired for an optical article with photochromic properties described above, high coloring density when colored may be mentioned. With respect to this point, a photochromic compound showing a high molar extinction coefficient when colored by being irradiated with light within a wavelength range having photoresponsivity; that is, a photochromic compound with high absorbing strength, for example, can impart photochromic properties that can be colored with high density to an optical article, even if a small amount of such a photochromic compound is added, and enables color development with further higher density by adding more amount of such a photochromic compound.

An object of one aspect of the present invention is to provide an optical article containing a photochromic compound with high absorption strength.

### [Solution to Problem]

One aspect of the present invention relates to
an optical article including one or more photochromic compounds selected from the group consisting of a compound represented by general formula 1 below and a compound represented by general formula 2 below.

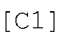

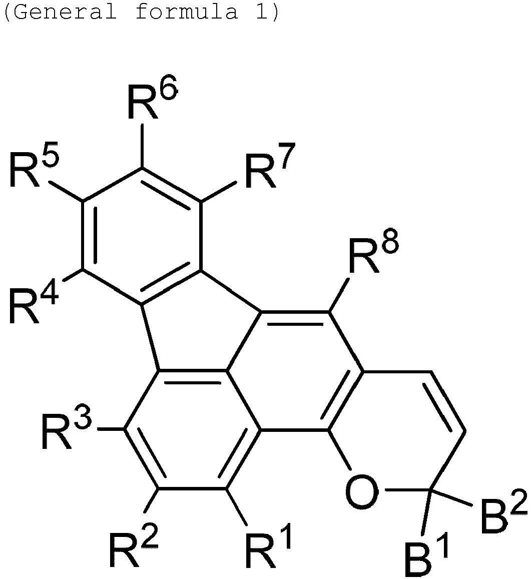

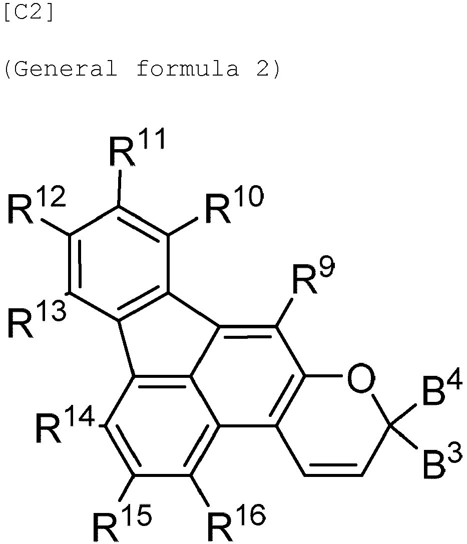

In the general formula 1, R¹ to R⁸, and B¹ and B² each independently represent a hydrogen atom or a substituent, and at least either of B¹ and B² represents a monovalent group represented by general formula 3 below;
in the general formula 2, R⁹ to R¹⁶, and B³ and B⁴ each independently represent a hydrogen atom or a substituent, and at least either of B³ and B⁴ represents a monovalent group represented by general formula 3 below;

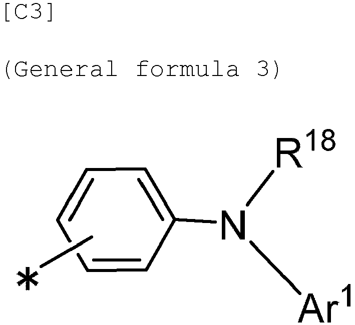

In the general formula 3, Ar¹ represents an aryl group or a heteroaryl group, R¹⁸ represents a hydrogen atom or a substituent, Ar¹ and R¹⁸ may be coupled to form a ring structure via a single bond or a divalent linking group, and * represents a bonding position to an adjacent carbon atom.

One aspect of the present invention relates to a photochromic compound represented by the general formula 1.

One aspect of the present invention relates to a photochromic compound represented by the general formula 2.

The photochromic compound represented by the general formula 1 and the photochromic compound represented by the general formula 2 show high molar extinction coefficients when colored by being irradiated with light within a wavelength range having photoresponsivity.

### [Advantageous Effects of Invention]

According to one aspect of the present invention, an optical article containing a photochromic compound with high absorption strength can be provided.

### [Brief Description of Drawings]

[Fig. 1]

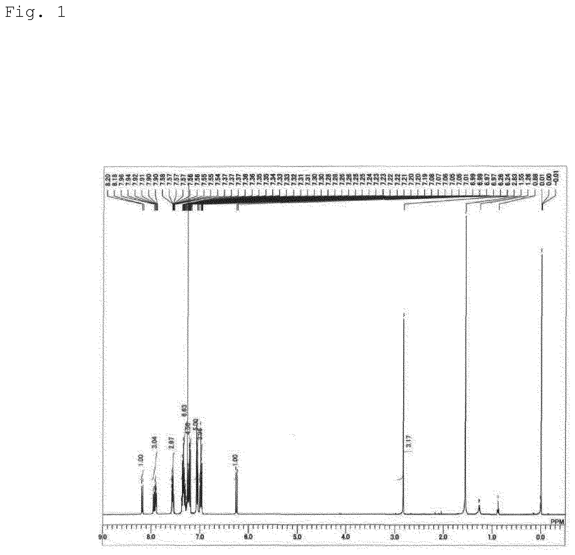

   Fig. 1 is an NMR chart of a compound in Example 1 (compound A) .
[Fig. 2]

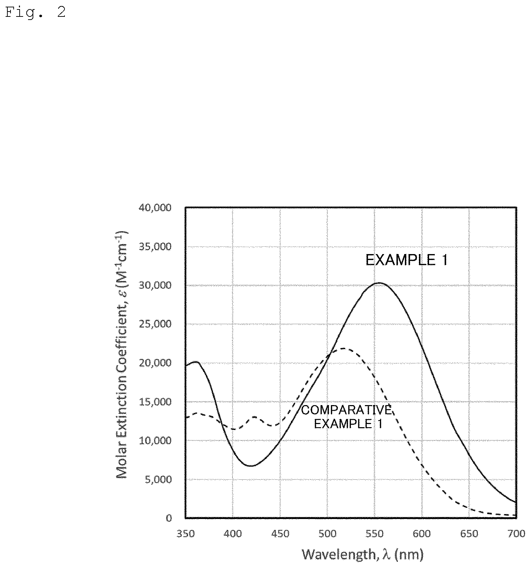

   Fig. 2 is a solution spectrum of Example 1 and Comparative Example 1 measured and obtained within 10 seconds from the end of the irradiation with UV light.

### [Description of Embodiments]

Hereinafter, the optical article and the photochromic compound will be described in more detail.

As one example, a photochromic compound undergoes an excited state by being irradiated with light, such as sunlight, and is structurally converted into a colored body. The structure after structural conversion via irradiation with light can be referred to as a "colored body". In contrast, the structure before irradiation with light can be referred to as a "colorless body". Note that the term "colorless" of a colorless body does not limitedly mean a complete colorless state and encompasses a state where the color is pale compared to the colored body. The structures of the general formula 1 and the general formula 2 are each a structure of a colorless body.

In the present invention and the present description, various substituents, that is, a substituent represented by any one of R¹ to R⁸, and B¹ and B² in the general formula 1, a substituent represented by any one of R⁹ to R¹⁶, and B³ and B⁴ in the general formula 2, a substituent represented by R¹⁸ in the general formula 3, and a substituent represented by any one of R¹⁹ to R²³ in the general formula 4, and further, a substituent when each group mentioned below has a substituent may each independently be a substituent R^{m}.

The substituent R^{m} is
a substituent selected from the group consisting of a hydroxy group; a linear or branched alkyl group with a carbon number of 1 to 18, such as a methyl group, an ethyl group, a propyl group, a butyl group, a pentyl group, or a hexyl group; a single-ring cycloaliphatic alkyl group with a carbon number of 5 to 18, such as a cyclopentyl group or a cyclohexyl group or a multiple-ring cycloaliphatic alkyl group with a carbon number of 5 to 18, such as a bicyclo ring; a linear or branched alkoxy group with the number of constituting atoms of 1 to 24, such as a methoxy group, an ethoxy group, or a butoxy group; a nonaromatic cyclic substituent with the number of constituting atoms of 1 to 24; a linear or branched perfluoroalkyl group with a carbon number of 1 to 18, such as a trifluoromethyl group; a linear or branched perfluoroalkoxy group such as a trifluoromethoxy group; a linear or branched alkyl sulfide group with the number of constituting atoms of 1 to 24, such as a methyl sulfide group, an ethyl sulfide group, or a butyl sulfide group; an aryl group such as a phenyl group, a naphthyl group, an anthracenyl group, fluoranthenyl group, a phenanthryl group, a pyranyl group, a perylenyl group, a styryl group, or a fluorenyl group; an aryloxy group such as a phenyloxy group; an aryl sulfide group such as a phenyl sulfide group; a heteroaryl group such as a pyridyl group, a furanyl group, a thienyl group, a pyrrolyl group, a benzofuranyl group, a benzothiophenyl group, an indolyl group, a dibenzofuranyl group, a dibenzothiophenyl group, a carbazolyl group, a diazolyl group, a triazolyl group, a quinolinyl group, a phenothiazinyl group, a phenoxazinyl group, a phenadinyl group, a thianthryl group, or an acridinyl group; an amino group (-NH₂); a monoalkylamino group such as a monomethylamino group; a dialkylamino group such as a dimethylamino group; a monoaryl amino group such as a monophenylamino group; a diarylamino group such as a diphenylamino group; a cyclic amino group such as a piperidino group, a morpholino group, a thiomorpholino group, a tetrahydroquinolino group, or a tetrahydroisoquinolino group; an ethynyl group; a mercapto group; a silyl group; a sulfonic acid group, an alkylsulfonyl group, a formyl group; a carboxy group; a cyano group; and a halogen atom such as a fluorine atom, a chlorine atom, a bromine atom, or an iodine atom; or
a substituent in which R^{m} is further substituted with one or more same or different R^{m}s.

As one example of the substituent in which R^{m} is further substituted with one or more same or different R^{m}s, a structure in which a terminal carbon atom of an alkoxy group is further substituted with an alkoxy group, and a terminal carbon atom of this alkoxy group is further substituted with another alkoxy group may be mentioned. As another example of the substituent in which R^{m} is further substituted with one or more same or different R^{m}s, a structure in which two or more positions among five substitutable positions in a phenyl group are substituted with the same or different R^{m}s may be mentioned. However, the present invention is not limited to these examples.

In the present invention and the present description, various substituents, that is, substituents represented by any one of R¹ to R⁸, and B¹ and B² in the general formula 1, a substituent represented by any one of R⁹ to R¹⁶, and B³ and B⁴ in the general formula 2, and a substituent represented by R¹⁸ in the general formula 3, and a substituent when each group mentioned below has a substituent may be solubilizing groups. In the present invention and the present description, a "solubilizing group" refers to a substituent that can contribute to increasing compatibility with any liquid or a specific liquid. Suitable solubilizing groups are substituents that can contribute to promoting the thermal motion of the molecules of a compound, such as an alkyl group with a carbon number of 4 to 50 with a linear, branched, or cyclic structure, a linear, branched, or cyclic alkoxy group with a constitution atom number of 4 to 50, a linear, branched, or cyclic silyl group with a constitution atom number of 4 to 50, a substituent in which a part of the group listed above is substituted with a silicon atom, a sulfur atom, a nitrogen atom, a phosphorus atom, or the like, or a combination of two or more of these groups listed above. A compound with a solubilizing group as a substituent can prevent the solidification of solutes by inhibiting the distance between the solute molecules from approaching each other and can create a molecular assembly state close to a liquid by lowering the melting point and/or glass transition temperature of solutes. In this way, a soluble group can liquidize a solute or can increase the solubility of a compound with this substituent in liquid. In one embodiment, as the solubilizing group, a linear alkyl group such as an n-butyl group, an n-pentyl group, an n-hexyl group, and an n-octyl group, a branched alkyl group, such as a tert-butyl group, and a cyclic alkyl group, such as a cyclopentyl group and a cyclohexyl group, are preferred.

The optical article may contain one or more compounds represented by the general formula 1, and may contain one or more compounds represented by the general formula 2. The optical article may contain only one compound represented by the general formula 1 or may contain two or more such compounds. The optical article may contain only one compound represented by the general formula 2 or may contain two or more such compounds. Alternatively, the optical article may contain only compounds represented by the general formula 1 or only compounds represented by the general formula 2 among the compounds represented by the general formula 1 and the compounds represented by the general formula 2, or may contain one or more compounds represented by the general formula 1 and one or more compounds represented by the general formula 2.

Hereinafter, the compound mentioned above will be described in more detail.

### [Photochromic Compound Represented by General Formula 1]

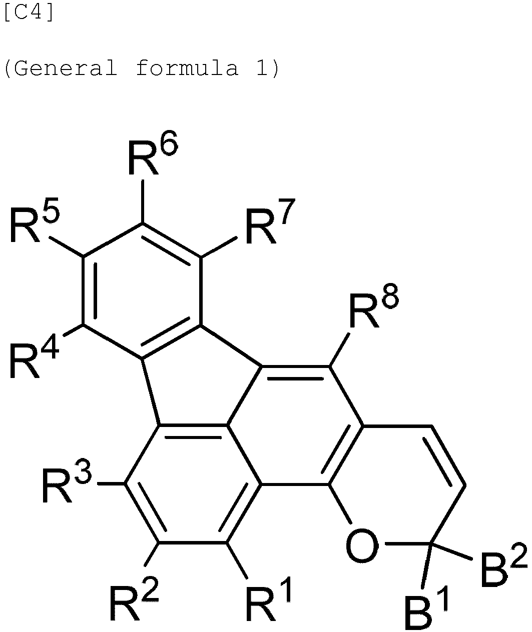

In the general formula 1, R¹ to R⁸, and B¹ and B² each independently represent a hydrogen atom or a substituent, and at least either of B¹ and B² represents a monovalent group represented by the following general formula 3. The present inventor infers that the presence of a monovalent group represented by the general formula 3 described below as at least one of B¹ and B² in the compound represented by the general formula 1 contributes to being able to exhibit a high molar absorption coefficient. The same applies to a case where the compound represented by the general formula 2 described below has a monovalent group represented by the general formula 3 described below as at least one of B³ and B⁴.

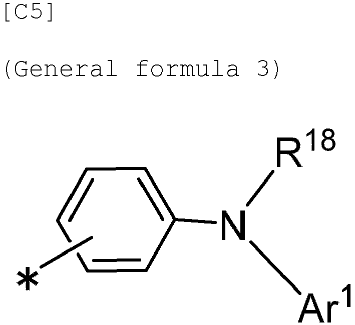

In the general formula 3, Ar¹ represents an aryl group or a heteroaryl group, R¹⁸ represents a hydrogen atom or a substituent, Ar¹ and R¹⁸ may be coupled to form a ring structure via a single bond or a divalent linking group, and * represents a bonding position to an adjacent carbon atom.

The monovalent group represented by the general formula 3 can be bonded to an adjacent carbon atom at any position of * and preferably bonded at a para position to the nitrogen atom at which Ar¹ and R¹⁸ are coupled. That is, the general formula 3 is preferably the following general formula 3-1.

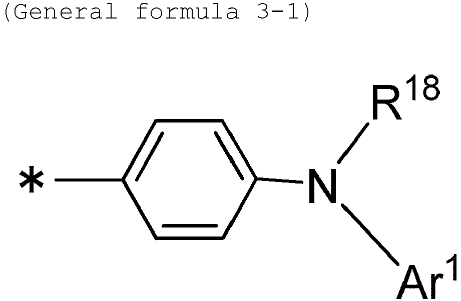

The aryl group represented by Ar¹ is a monovalent group with an aromatic ring, and the carbon number constituting such an aromatic ring is, for example, 6 to 60. The heteroaryl group represented by Ar¹ is a monovalent group with a heteroaromatic ring, and the carbon number constituting such a heteroaromatic ring is, for example, 5 to 60, and at least one of them is a heteroatom. For example, the heteroatom is a nitrogen atom, an oxygen atom, and a sulfur atom.

Specific examples of the aromatic and heteroaromatic rings are listed below. The aromatic ring and the heteroaromatic ring listed below may be unsubstituted or have any number of substituents at any position.

A benzene ring, a naphthalene ring, an anthracene ring, a benzanthracene ring, a phenanthrene ring, a benzphenanthrene ring, a pyrene ring, a chrysene ring, a perylene ring, a fluoranthene ring, a naphthacene ring, a pentacene ring, a benzopyrene ring, a biphenyl ring, a biphenylene ring, a terphenyl ring, a terphenylene ring, a quaterphenyl ring, a fluorene ring, a spirobifluorene ring, a dihydrophenanthrene ring, a dihydropyrene ring, a tetrahydropyrene ring, a cis- or trans-indenofluorene ring, a torquekin ring, an isotruxene ring, a spirotruxene ring, a spiroisotruxene ring, a furan ring, a benzofuran ring, an isobenzofuran ring, a dibenzofuran ring, a thiophene ring, a benzothiophene ring, an isobenzothiophene ring, a dibenzothiophene ring, a pyrrole ring, an indole ring, an isoindole ring, a carbazole ring, an indolocarbazole ring, an indenocarbazole ring, a pyridine ring, a quinoline ring, an isoquinoline ring, an acridine ring, a phenanthridine ring, a benzo-5,6-quinoline ring, a benzo-6,7-quinoline ring, a benzo-7,8-quinoline ring, a phenothiazine ring, a phenoxazine ring, a pyrazole ring, an indazole ring, an imidazole ring, an benzimidazole ring, a naphthoimidazole ring, a phenanthrimidazol ring, a pyridoimidazole ring, a pyrazinimidazol ring, a quinoxalineimidazol ring, an oxazole ring, a benzoxazole ring, a naphthoxazole ring, an anthrooxazole ring, a phenanthrooxazole ring, an isooxazole ring, a 1,2-thiazole ring, a 1,3-thiazole ring, a benzothiazole ring, a pyridazine ring, a benzopyridazine ring, a pyrimidine ring, a benzopyrimidine ring, a quinoxaline ring, a 1,5-diazaanthracene ring, a 2,7-diazapyrene ring, a 2,3-diazapyrene ring, a 1,6-diazapyrene ring, a 1,8-diazapyrene ring, a 4,5-diazapyrene ring, a 4,5,9,10-tetraazaperylene ring, a radine ring, a phenazine ring, a fluorbine ring, a naphthyridine ring, an azacarbazole ring, a benzocarboline ring, a phenanthroline ring, a 1,2,3-triazole ring, a 1,2,4-triazole ring, a benzotriazole ring, a 1,2,3-oxadiazole ring, a 1,2,4-oxadiazole ring, a 1,2,5-oxadiazole ring, a 1,3,4-oxadiazole ring, a 1,2,3-thiadiazole ring, a 1,2,4-thiadiazole ring, a 1,2,5-thiadiazole ring, a 1,3,4-thiadiazole ring, a 1,3,5-triazine ring, a 1,2,4-triazine ring, a 1,2,3-triazine ring, a tetrazole ring, a 1,2,4,5-tetrazine ring, a 1,2,3,4-tetrazine ring, a 1,2,3,5-tetrazine ring, a purine ring, a pteridine ring, an indolizine ring, and a benzothiadiazole ring; and an aromatic and heteroaromatic ring in which two or more kinds selected from the group consisting of the above aromatic rings and heterocyclic aromatic rings are condensed.

Preferred aromatic and heteroaromatic rings among them are listed below.

A benzene ring, a naphthalene ring, a phenanthrene ring, a fluoranthene ring, a biphenyl ring, a fluorene ring, a dibenzofuran ring, a dibenzothiophene ring, a carbazole ring, an acridine ring, a phenothiazine ring, a phenoxazine ring, a phenazine ring, an azacarbazole ring, and a phenanthroline ring; and an aromatic and heteroaromatic ring in which two or more kinds selected from the group consisting of the above aromatic rings and heterocyclic aromatic rings are condensed.

More preferred aromatic and heteroaromatic rings among them are listed below.

A benzene ring, a biphenyl ring, a fluorene ring, a dibenzofuran ring, and a phenanthroline ring,; and an aromatic and heteroaromatic ring in which two or more kinds selected from the group consisting of the above aromatic rings and heterocyclic aromatic rings are condensed.

Ar¹ preferably represents an aryl group and more preferably represents a substituted or unsubstituted phenyl group, a substituted or unsubstituted biphenyl group, or a substituted or unsubstituted fluorenyl group.

For example, when a compound represented by the general formula 1 is irradiated with light within a wavelength range having photoresponsivity (for example, UV ray) and colored, the maximum absorption wavelength of the compound within the wavelength range of 400 nm or more may be 540 to 560 nm. A photochromic compound with such absorption characteristics is preferable in that it can effectively absorb the light around 555 nm, which is the maximum value of relative luminosity in brightfield vision, and therefore, luminous transmittance when colored tends to be lower. From this point, it is preferred that Ar¹ is one of the various groups mentioned above. The same applies to the compound represented by the general formula 2.

In the general formula 3, R¹⁸ represents a hydrogen atom or a substituent. As the substituent represented by R¹⁸, an aryl group or a heteroaryl group may be mentioned. Regarding these groups, reference can be made to the earlier description about aryl groups or heteroaryl groups represented by Ar¹. R¹⁸ preferably represents an aryl group and more preferably represents a substituted or unsubstituted phenyl group, a substituted or unsubstituted biphenyl group, or a substituted or unsubstituted fluorenyl group.

The aryl group or heteroaryl group represented by Ar¹ may be coupled to a benzene ring bonded to the nitrogen atom in the general formula 3 via a single bond or a divalent linking group to form a ring structure.

When the single bond or a divalent linking group is represented as L¹, the monovalent group represented by the general formula 3 may be a monovalent group represented by the following general formula 3-2 or the following general formula 3-3. In the general formula shown below, Ar¹, R¹⁸, and * are respectively as defined in the general formula 3.

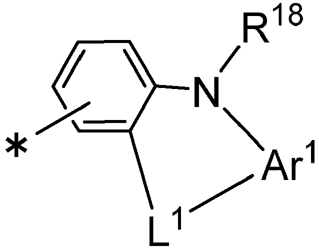

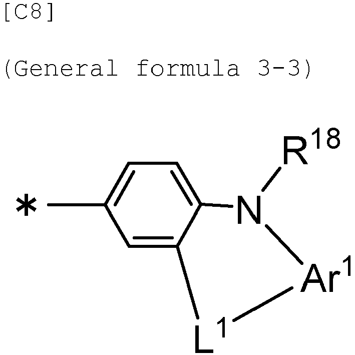

L¹ may be a single bond or a divalent linking group selected from the group consisting of a substituted or unsubstituted linear, branched, or cyclic alkylene group with a carbon number of 1 to 10 (preferably, 2 or less), an arylene group (preferably, phenylene group) with a carbon number of 1 to 20, and an alkylarylene group with a carbon number of 1 to 20. As the divalent linking group represented by L¹, a divalent linking group in which part of carbon atoms constituting the divalent linking group listed above is substituted with other atoms, such as nitrogen atoms, oxygen atoms, sulfur atoms, or silicon atoms. As such a group, for example, a divalent linking group such as an ether group (-O-), a sulfide group(-S-), -NR¹⁰⁰-, or the like may be mentioned. R¹⁰⁰ represents a hydrogen atom or a substituent, and such a substituent is, for example, an alkyl group, preferably a substituted or unsubstituted linear, branched, or cyclic alkyl group with a carbon number of 1 to 10 (preferably, 2 or less).

The substituent represented by R¹⁸ may be coupled to a benzene ring bonded to the nitrogen atom in the general formula 3 via a single bond or a divalent linking group to form a ring structure.

When the single bond or a divalent linking group is represented as L³, the monovalent group represented by the general formula 3 may be a monovalent group represented by general formula 3-4 or general formula 3-5 below. In the general formula shown below, Ar¹, R¹⁸, and * are respectively as defined in the general formula 3. For L³, reference can be made to the earlier description about L¹.

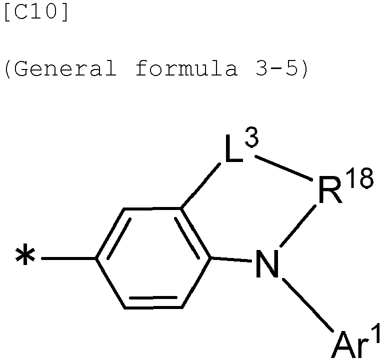

In the general formula 3, Ar¹ and R¹⁸ may be coupled together via a single bond or a divalent linking group to form a ring structure. When the single bond or a divalent linking group is represented as L², the monovalent group represented by the general formula 3 may be a monovalent group represented by general formula 3-6 or general formula 3-7 below. In the general formula shown below, Ar¹, R¹⁸, and * are respectively as defined in the general formula 3. For L², reference can be made to the earlier description about L¹.

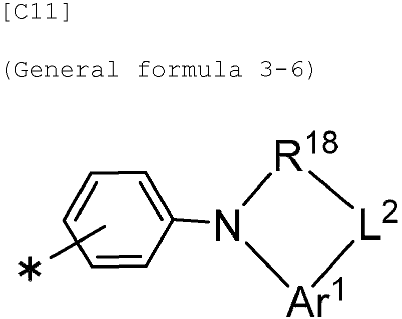

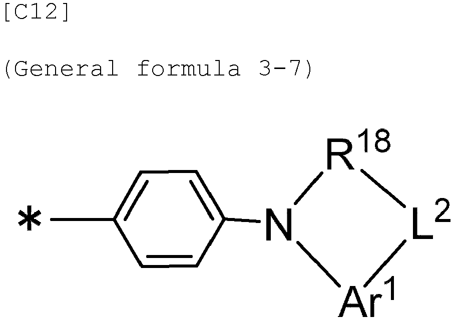

As specific examples of ring structures formed when Ar¹ and R¹⁸ are coupled together via a single bond or a divalent linking group, various ring structures included in the compounds listed below may be mentioned.

In general formula 1, at least one of B¹ and B² represents a monovalent represented by general formula 3. Among B¹ and B², only one of them can represent a monovalent group represented by the general formula 3 in one embodiment, or both of them can each independently represent a monovalent group represented by the general formula 3 in another embodiment. Preferably, only one of B¹ and B² represents a monovalent group represented by the general formula 3, and the other is a hydrogen atom or a substituent other than the monovalent group represented by the general formula 3. As such substituents, a monovalent group represented by the general formula 4 below may be mentioned.

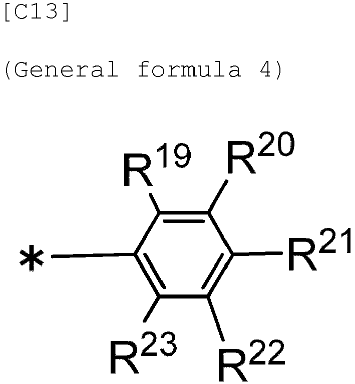

In the general formula 4, R¹⁹ to R²³ each independently represent a hydrogen atom or a substituent.

In the general formula 1, R¹ to R⁸ each independently represent a hydrogen atom or a substituent.

As substituents that can be encompassed in the group listed earlier as B¹ or B² or substituents that can be represented by B¹ or B²,
a propyl group, a butyl group, a hexyl group, a cyclohexyl group, a methoxy group, an ethoxy group, a phenoxy group, a methyl sulfide group, an ethyl sulfide group, a phenyl sulfide group, a trifluoromethyl group, a phenyl group, a naphthyl group, a dibenzofuranyl group, a dibenzothiophenyl group, a carbazolyl group, a phenothiazinyl group, a phenoxazinyl group, a phenazinyl group, an acridinyl group, a dimethylamino group, a diphenylamino group, a piperidino group, a morpholino group, a thiomorpholino group, a cyano group, a fluorine atom, and a solubilizing group are preferred, and
a methoxy group, a phenoxy group, a methyl sulfide group, a phenyl sulfide group, a trifluoromethyl group, a phenyl group, a dimethylamino group, a diphenyl amino group, a piperidino group, a morpholino group, a thiomorpholino group, a cyano group, a fluorine atom, and a solubilizing group are more preferred.

As preferable substituents as any one of R¹ to R⁸, a methyl group, an ethyl group, a propyl group, a butyl group, a hexyl group, a cyclopentyl group, a cyclohexyl group, a methoxy group, a phenoxy group, a methyl sulfide group, a phenyl sulfide group, a trifluoromethyl group, a phenyl group, a dimethylamino group, a diphenylamino group, a piperidino group, a morpholino group, a thiomorpholino group, a cyano group, a fluorine atom, and a solubilizing group may be mentioned.

In one embodiment, among R¹ to R⁸, R⁸ represents a substituent and the others represent hydrogen atoms. In another embodiment, among R¹ to R⁸, R⁸, and R¹ and/or R⁶ represent substituents and the others represent hydrogen atoms.

As preferable substituents as R⁸, a linear or branched alkyl group with a carbon number of 1 to 18 may be mentioned, and a methyl group, an ethyl group, a propyl group, a butyl group, or a hexyl group are preferred.

As preferable substituents as R⁶, a methyl group, a trifluoromethyl group, and a fluorine atom may be mentioned.

As preferable substituents as R¹, a methyl group, a trifluoromethyl group, and a fluorine atom may be mentioned.

Two or more of R¹ to R⁸ may be coupled together via a single bond or a divalent linking group to form a ring structure.

As preferable substituents as any one of R¹⁹ to R²³, a hydroxy group; a linear or branched alkyl group with a carbon number of 1 to 18; a single ring or multiple ring cyclic aliphatic alkyl group with a carbon number of 5 to 18; a linear or branched alkoxy group with a constitution atom number of 1 to 24, such as a methoxy group, an ethoxy group, and a butoxy group; a linear or branched alkyl sulfide group with a constitution atom number of 1 to 24, such as a methyl sulfide group, an ethyl sulfide group, and a butyl sulfide group; an aryloxy group such as a phenyloxy group; an arylsulfide group such as a phenylsulfide group; a dialkylamino group such as dimethylamino group; a piperidino group; a morpholino group; and fluorine atom may be mentioned, and a substituent in which R^{m} is further substituted with one or more same or different R^{m}s, for example, substituents described below also may be mentioned. Hereinafter, * represents a bonding position with an adjacent carbon atom. In one embodiment, among R¹⁹ to R²³, it is preferred that at least R²¹ is a substituent, and it is more preferred that R²¹ is a substituent, and the others are hydrogen atoms. In another embodiment, all of the R¹⁹ to R²³ are preferably hydrogen atoms.

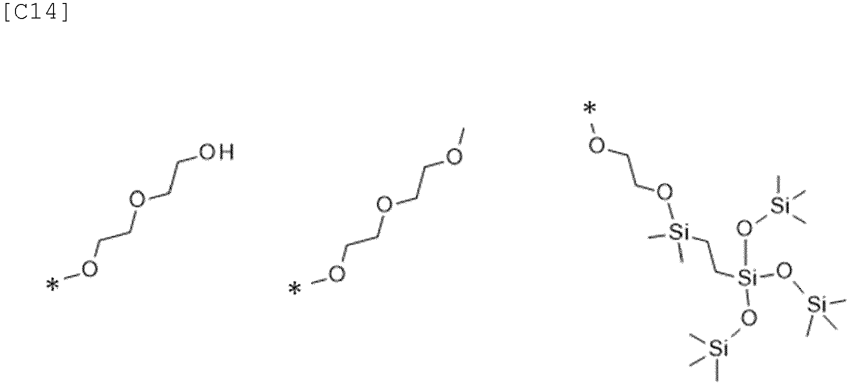

### [Photochromic Compound Represented by General Formula 2]

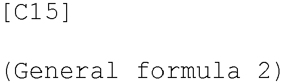

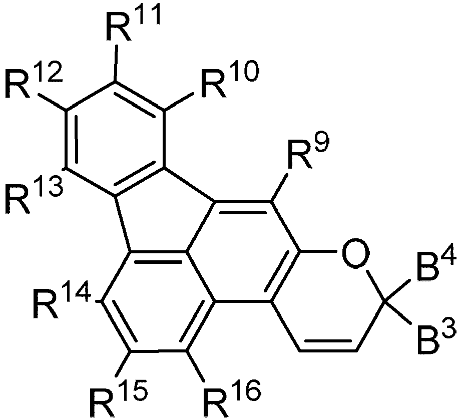

In the general formula 2, R⁹ to R¹⁶, and B³ and B⁴ each independently represent a hydrogen atom or a substituent, and at least either of B³ and B⁴ represents a monovalent group represented by the general formula 3 mentioned above. The general formula 3 is as described above.

In the general formula 2, at least one of B³ and B⁴ represents a monovalent represented by the general formula 3. Among B³ and B⁴, only one of them can represent a monovalent group represented by the general formula 3 in one embodiment, or both of them can each independently represent a monovalent group represented by the general formula 3 in another embodiment. Preferably, only one of B³ and B⁴ represents a monovalent group represented by the general formula 3, and the other is a hydrogen atom or a substituent other than the monovalent group represented by the general formula 3. As such a substituent, a monovalent group represented by the general formula 4 mentioned above may be mentioned. The general formula 4 is as described above.

As substituents that can be encompassed in the group listed earlier as B³ or B⁴ or substituents that can be represented by B³ or B⁴,
a propyl group, a butyl group, a hexyl group, a cyclohexyl group, a methoxy group, an ethoxy group, a phenoxy group, a methyl sulfide group, an ethyl sulfide group, a phenyl sulfide group, a trifluoromethyl group, a phenyl group, a naphthyl group, a dibenzofuranyl group, a dibenzothiophenyl group, a carbazolyl group, a phenothiazinyl group, a phenoxazinyl group, a phenazinyl group, an acridinyl group, a dimethylamino group, a diphenylamino group, a piperidino group, a morpholino group, a thiomorpholino group, a cyano group, a fluorine atom, and a solubilizing group are preferred, and
a methoxy group, a phenoxy group, a methyl sulfide group, a phenyl sulfide group, a trifluoromethyl group, a phenyl group, a dimethylamino group, a diphenyl amino group, a piperidino group, a morpholino group, a thiomorpholino group, a cyano group, a fluorine atom, and a solubilizing group are more preferred.

As preferable substituents as any one of R⁹ to R¹⁶, a methyl group, an ethyl group, a propyl group, a butyl group, a hexyl group, a cyclopentyl group, a cyclohexyl group, a methoxy group, a phenoxy group, a methyl sulfide group, a phenyl sulfide group, a trifluoromethyl group, a phenyl group, a dimethylamino group, a diphenylamino group, a piperidino group, a morpholino group, a thiomorpholino group, a cyano group, a fluorine atom, and a solubilizing group may be mentioned.

In one embodiment, among R⁹ to R¹⁶, R⁹ represents a substituent and the others represent hydrogen atoms. In another embodiment, among R⁹ to R¹⁶, R⁹, and R¹¹ and/or R¹⁶ represent substituents and the others represent hydrogen atoms. As preferable substituents as R⁹, a linear or branched alkyl group with a carbon number of 1 to 18 may be mentioned, and a methyl group, an ethyl group, a propyl group, a butyl group, or a hexyl group are preferred.

As preferable substituents as R¹⁶, a methyl group, a trifluoromethyl group, and a fluorine atom may be mentioned.

As preferable substituents as R¹¹, a methyl group, a trifluoromethyl group, and a fluorine atom may be mentioned.

The photochromic compound represented by the general formula 1 and the photochromic compound represented by the general formula 2 may be used for constructing an article with photochromic properties (a photochromic article). As specific examples of such compounds, the following compounds can be listed. However, the present invention is not limited to the compounds listed below.

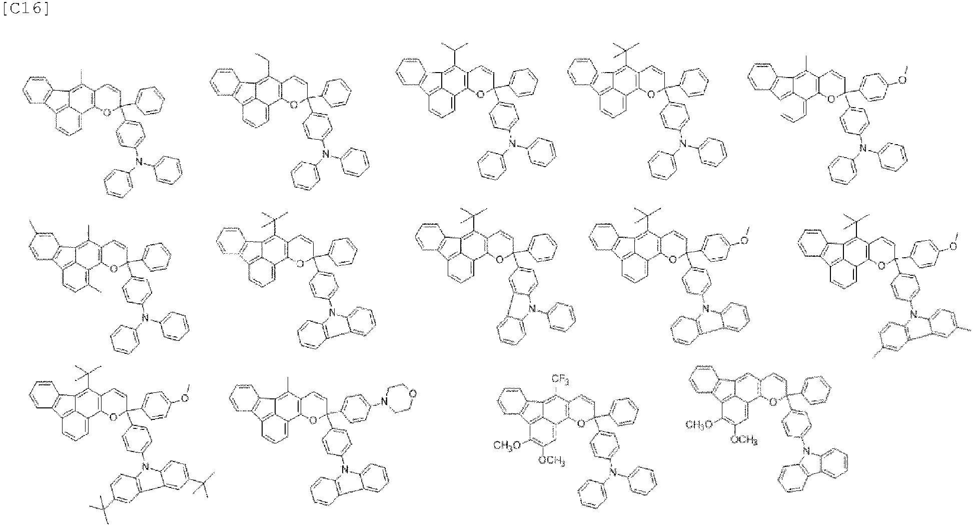

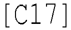

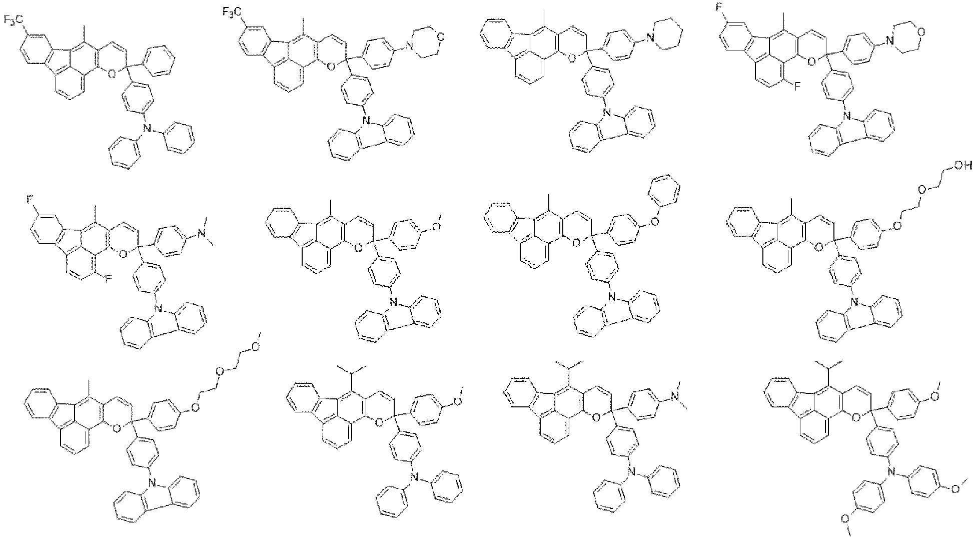

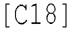

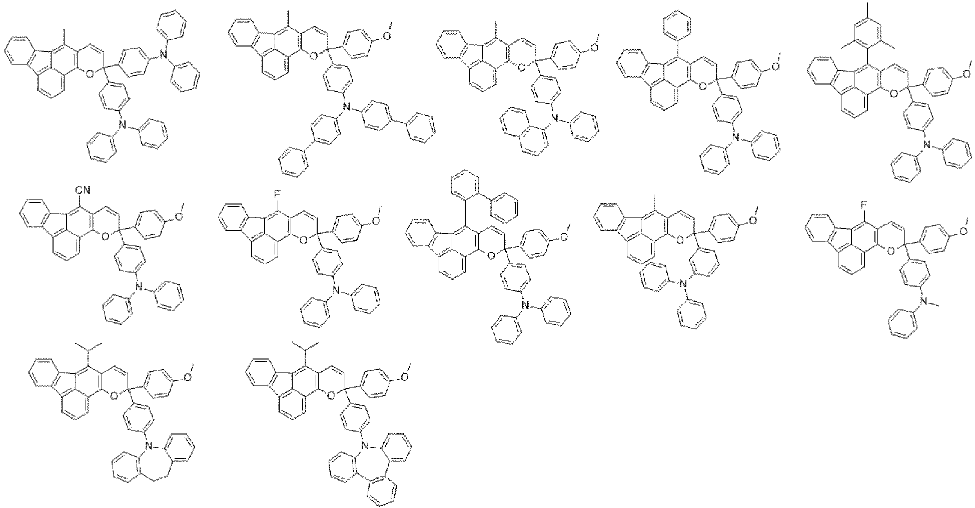

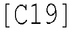

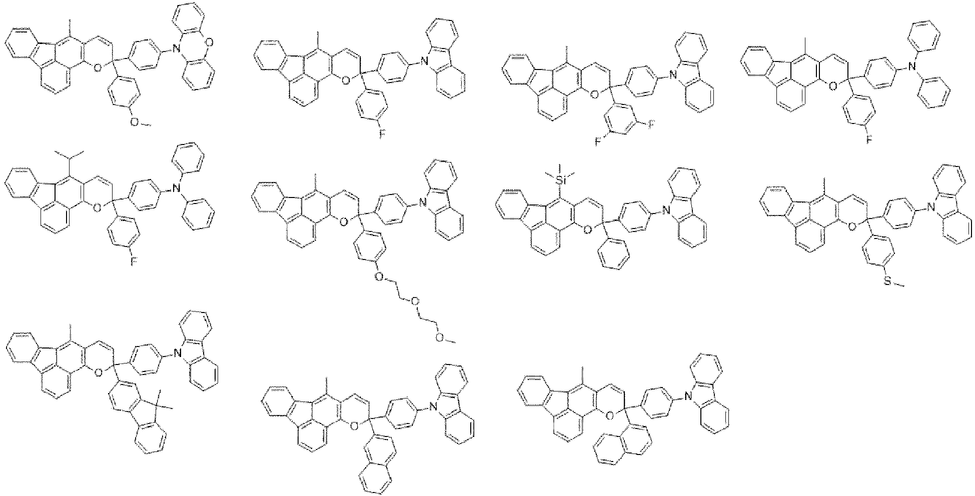

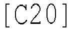

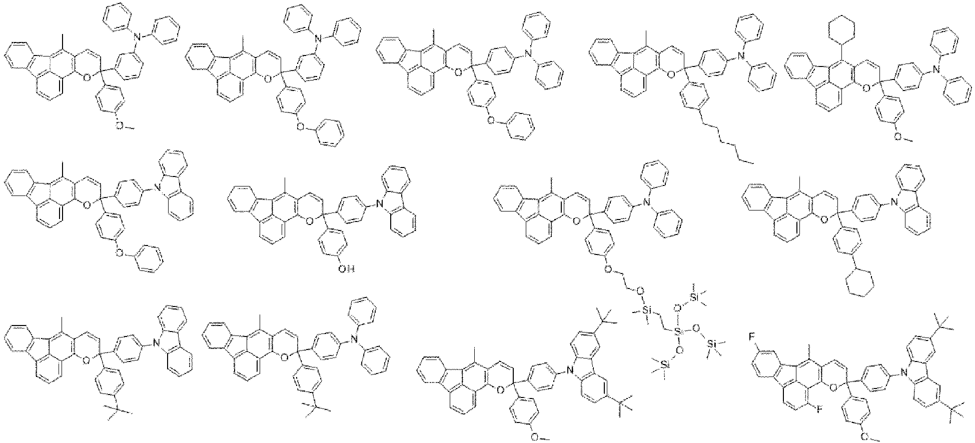

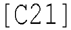

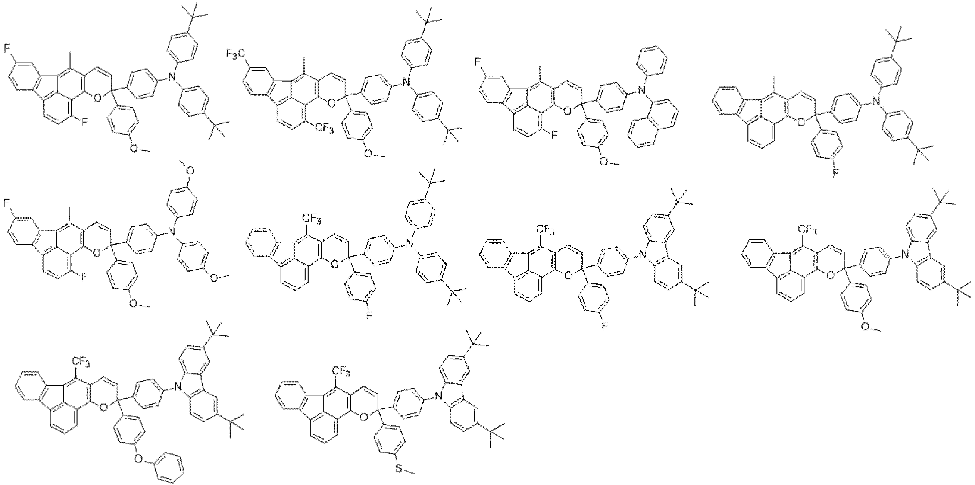

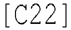

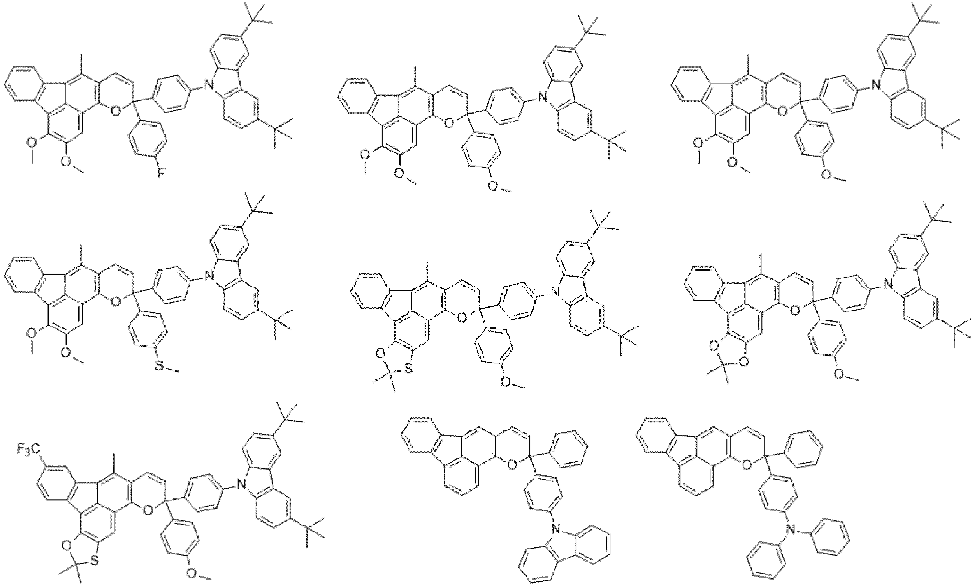

The compound represented by the general formula 1 and the compound represented by the general formula 2 may be synthesized by known methods.

For example, the compound represented by the general formula 1 may be synthesized by the reaction between the following naphthol derivative: 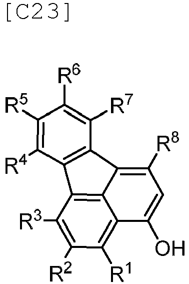 and the following propargyl alcohol derivative:

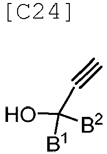

. The R¹ to R⁸ and B¹ and B² are as defined in the general formula 1.

In addition, for example, the compound represented by the general formula 2 may be synthesized by the reaction between the following naphthol derivative: 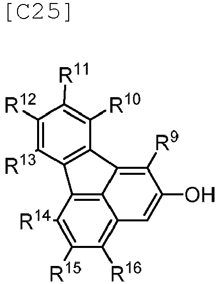 and the following propargyl alcohol derivative:

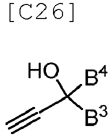

. The R⁹ to R¹⁶ and B³ and B⁴ are as defined in the general formula 2.

For details of the reaction conditions, etc., for synthesizing the compound represented by the general formula 1 and the compound represented by the general formula 2, known techniques can be applied, and the examples described below can also be referred to.

### [Optical Article]

The compound represented by the general formula 1 and the compound represented by the general formula 2 are both photochromic compounds. The photochromic compound may be incorporated in a substrate of an optical article, and/or in a photochromic layer of an optical article. The optical article may contain only one of the photochromic compounds in one embodiment or may contain two or more of the photochromic compounds in another embodiment. Alternatively, the optical article may contain one or more of the photochromic compounds and one or more other photochromic compounds in one embodiment. Examples of the other photochromic compounds may include azobenzenes, spiropyrans, spirooxazines, naphthopyrans, indenonaphthopyrans, phenanthropyrans, hexaallyl bisimidazoles, donor-acceptor Stenhouse adducts (DASA), salicylideneanilines, dihydropyrenes, anthracene dimers, fulgides, diarylethenes, phenoxynaphthacenequinones, stilbenes, and the like.

The optical article may include a substrate selected according to the type of optical article. As one example of a substrate, a plastic lens substrate or glass lens substrate as a spectacle lens substrate may be mentioned. For example, a glass lens substrate may be a lens substrate made of inorganic glass. Examples of a plastic lens substrate may include a styrene resin including a (meth)acrylate resin, a polycarbonate resin, an allylic resin, an allyl carbonate resin such as a diethylene glycol bis(allyl carbonate) resin (CR-39), a vinyl resin, a polyester resin, a polyether resin, a urethane resin obtained by a reaction between an isocyanate compound and a hydroxy compounds such as diethylene glycol, a thiourethane resin obtained by a reaction between an isocyanate compound and a polythiol compound, and a cured product (which is generally called transparent resins) of a curable composition containing a (thio)epoxy compound having one or more disulfide bonds in a molecule. As a lens substrate, an unstained substrate (colorless lens) may be used, and a stained substrate (stained lens) may be used. For example, the refractive index of the lens substrate may be around 1.60 to 1.75. However, the refractive index of the lens substrate is not limited to the above range and may be within the range or may alternatively be over or below the range. The refractive index shall herein mean a refractive index for the light of wavelength 500 nm. The lens substrate may be a lens with refractive power (so-called prescription lens) or may be a lens without refractive power (so-called non-prescription lens) .

For example, a substrate can be prepared by molding a curable composition containing one or more of the photochromic compounds mentioned above and one or more polymerizable compounds by a known method to prepare the substrate as a cured product of such a curable composition. The curable composition may further contain one or more other photochromic compounds listed earlier. The curing treatment may be light irradiation and/or heating treatment. A polymerizable compound is a compound with a polymerizable group, and a curable composition can be cured as the progress of the polymerization reaction of the polymerizable compound to form a curable product. The curable compositions may further contain one or more additives (for example, a polymerization initiator).

The spectacle lens may include various lenses such as a single-focus lens, a multifocal lens, and a progressive power lens. The surface shape of both sides of a lens substrate determines the lens type. In addition, the surface of the lens substrate may be convex, concave, or flat. In ordinary lens substrates and spectacle lenses, the object-side surface is convex, and the eyeball-side surface is concave. However, the present invention is not limited thereto. The photochromic layer may be generally provided on the object-side surface of the lens substrate, but may be provided on the eyeball-side surface.

A photochromic layer may be a layer directly formed on the surface of the substrate or indirectly formed via one or more other layers. For example, the photochromic layer may be a cured layer formed by curing a curable composition containing the photochromic compound mentioned above. The curable compositions may further contain one or more other photochromic compounds listed earlier. For example, a cured layer containing the photochromic compound may be formed by applying the curable composition directly on the surface of a substrate, or applying the curable composition on the surface of a layer formed on a substrate, and then curing the applied curable composition. For an application method, known application methods such as a spin coating method or a dip coating method may be adopted. The curing treatment may be light irradiation and/or heating treatment. The curable compositions may contain a polymerizable compound and may further contain one or more additives (e.g., a polymerization initiator) The curable composition can be cured as the progress of the polymerization reaction of the polymerizable compound to form a cured layer.

### <Polymerizable Compound>

In the present invention and the present description, a polymerizable compound shall refer to a compound with one or more polymerizable groups in a molecule, and a "polymerizable group" shall refer to a reactive group that can cause a polymerization reaction. Examples of polymerizable groups may include an acryloyl group, a methacryloyl group, a vinyl group, a vinyl ether group, an epoxy group, a thiol group, an oxetane group, a hydroxy group, a carboxy group, an amino group, an isocyanate group, and the like.

As examples of polymerizable compounds available for forming the above substrate and the above photochromic layer, the following compounds may be mentioned.

### (Episulfide-based Compound)

An episulfide-based compound is a compound with two or more episulfide groups in one molecule. An episulfide group is a polymerizable group that can cause ring-opening polymerization. Specific examples of episulfide compounds may include bis(1,2-epithioethyl)sulfide, bis(1,2-epithioethyl)disulfide, bis(2,3-epithiopropyl)sulfide, bis(2,3-epithiopropylthio)methane, bis(2,3-epithiopropyl)disulfide, bis(2,3-epithiopropyldithio)methane, bis(2,3-epithiopropyldithio)ethane, bis(6,7-epithio-3,4-dithiaheptyl)sulfide, bis(6,7-epithio-3,4-dithiaheptyl)disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithietane, 2-[1,1-bis(2,3-epithiopropyldithiomethylthio)methyl]-1,3-dithietane, and the like.

### (Thietanyl-based Compound)

A thietanyl compound is a thietane compound with two or more thietanyl groups in one molecule. A thietanyl group is a polymerizable group that can cause ring-opening polymerization. Some thietanyl compounds have an episulfide group with a plurality of thietanyl groups. Such compounds are listed in the examples of the episulfide compounds mentioned above. Other thietanyl compounds include metal-containing thietane compounds that contain metal atoms in the molecule and non-metallic thietane compounds that do not contain any metal.

Specific examples of non-metallic thietane compounds may include bis(3-thietanyl) disulfide, bis(3-thietanyl) sulfide, bis(3-thietanyl) trisulfide, bis(3-thietanyl) tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiaundecane, 1,11-bis(3-thietanylthio)-4,7-bis(3-thietanylthiomethyl)-3,6,9-trithiaundecane, 1,11-bis(3-thietanylthio)-5,7-bis(3-thietanylthiomethyl)-3,6,9-trithiaundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bisthietanyl sulfide, bis(thietanylthio)methane, 3-[<(thietanylthio)methylthio>methylthio]thietane, bisthietanyl disulfide, bisthietanyl trisulfide, bisthietanyl tetrasulfide, bisthietanyl pentasulfide, 1,4-bis(3-thietanyldithio)-2,3-dithiabutane, 1,1,1-tris(3-thietanyldithio)methane, 1,1,1-tris(3-thietanyldithiomethylthio)methane, 1,1,2,2-tetrakis(3-thietanyldithio)ethane, 1,1,2,2-tetrakis(3-thietanyldithiomethylthio)ethane, and the like.

Examples of metal-containing thietane compounds may include compounds containing, as metal atoms, in a molecule, group 14 atoms such as an Sn atom, a Si atom, a Ge atom, and a Pb atom, a group 4 atoms such as a Zr atom and a Ti atom, 13 group atoms such as an Al atom, 12 group atoms such a Zn atom, and the like. Specific examples may include alkylthio(thietanylthio)tins, bis(alkylthio)bis (thietanylthio)tins, alkylthio(alkylthio)bis(thietanylthio)tins, bis(thietanylthio)cyclic dithiotin compounds, alkyl(thietanylthio)tin compounds, and the like.

Specific examples of alkylthio(thietanylthio)tins may include methylthiotris(thietanylthio)tin, ethylthiotris(thietanylthio)tin, propylthiotris(thietanylthio)tin, isopropylthiotris(thietanylthio)tin, and the like.

Specific examples of bis(alkylthio)bis(thietanylthio)tins may include bis(methylthio)bis(thietanylthio)tin, bis(ethylthio)bis (thietanylthio)tin, bis(propylthio)bis (thietanylthio)tin, bis(isopropylthio)bis(thietanylthio)tin, and the like.

Specific examples of alkylthio(alkylthio)bis(thietanylthio)tins may include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, isopropylthio(propylthio)bis(thietanylthio)tin, and the like.

Specific example of bis(thietanylthio)cyclicdithiotin compounds may include bis(thietanylthio)dithiastannetane, bis(thietanylthio)dithiastannolane, bis(thietanylthio)dithiastanninane, bis(thietanylthio)trithiastannocane, and the like.

Specific examples of alkyl(thietanylthio)tin compounds may include methyltris(thietanylthio)tin, dimethylbis(thietanylthio)tin, butyltris(thietanylthio)tin, tetrakis(thietanylthio)tin, tetrakis(thietanylthio)germanium, tris(thietanylthio)bismuth, and the like.

### (Polyamine Component)

A polyamine compound is a compound with two or more NH₂ groups in a molecule, and can form a urea bond by the reaction with a polyisocyanate, and can form a thiourea bond by the reaction with a polyisothiocyanate. Specific examples of polyamine compounds may include ethylenediamine, hexamethylenediamine, isophoronediamine, nonamethylenediamine, undecamethylenediamine, dodecamethylenediamine, m-xylenediamine, 1,3-propanediamine, putrescine, 2-(2-aminoethylamino)ethanol, diethylenetriamine, p-phenylenediamine, m-phenylenediamine, melamine, 1,3,5-benzenetriamine, and the like.

### (Epoxy-based Compound)

An epoxy-based compound is a compound with an epoxy group in the molecule. An epoxy group is a polymerizable group that can cause ring-opening polymerization. Generally, epoxy-based compounds are classified into aliphatic epoxy compounds, alicyclic epoxy compounds, and aromatic epoxy compounds.

Specific examples of aliphatic epoxy compounds may include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentylglycol diglycidyl ether, trimethylolpropane triglycidyl ether, glycerol triglycidyl ether, diglycerol tetraglycidyl ether, pentaerythritol tetraglycidyl ether, triglycidyl ether of tris(2-hydroxyethyl) isocyanurate, and the like.

Specific examples of alicyclic epoxy compounds may include isophorone diol diglycidyl ether, bis-2,2-hydroxycyclohexylpropane diglycidyl ether, and the like.

Specific examples of aromatic epoxy compounds may include resorcin diglycidyl ether, bisphenol A diglycidyl ether, bisphenol F diglycidyl ether, bisphenol S diglycidyl ether, diglycidyl orthophthalate, phenol novolac polyglycidyl ether, cresol novolac polyglycidyl ether, and the like.

Besides the above, an epoxy-based compound with a sulfur atom in the molecule can be used together with an epoxy group. Such sulfur atom-containing epoxy-based compounds include chain aliphatic epoxy-based compounds and cyclic aliphatic epoxy-based compounds.

Specific examples of chain aliphatic sulfur atom-containing epoxy-based compounds may include bis(2,3-epoxypropyl)sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis(2,3-epoxypropylthiomethyl)propane, 2,2-bis(2,3-epoxypropylthiomethyl)-1-(2,3-epoxypropylthio)butane, and the like.

Specific examples of cyclic aliphatic sulfur atom-containing epoxy-based compounds may include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, 2,5-bis(2,3-epoxypropylthiomethyl)-2,5-dimethyl-1,4-dithiane, and the like.

### (Compound with a Radically Polymerizable Group)

A compound with a radically polymerizable group is a radically polymerizable group. Examples of radically polymerizable groups may include an acryloyl group, a methacryloyl group, an allyl group, a vinyl group, and the like.

Hereinafter, a compound with a polymerizable group selected from the group consisting of an acryloyl group and a methacryloyl group is referred to as a "(meth)acrylate compound". Specific examples of (meth)acrylate compounds may include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl (meth)acrylate, bisphenol A di(meth)acrylate, 2,2-bis(4-(meth)acryloxyethoxyphenyl)propane, 2,2-bis(4-(meth)acryloxydiethoxyphenyl)propane, 2,2-bis(4-(meth)acryloxyethoxyphenyl)propane, 2,2-bis(3,5-dibromo-4-(meth)acryloyloxyethoxyphenyl)propane, 2,2-bis(4-(meth)acryloyloxydipropoxyphenyl)propane, bisphenol F di(meth)acrylate, 1,1-bis(4-(meth)acryloxyethoxyphenyl)methane, 1,1-bis(4-(meth)acryloxydiethoxyphenyl)methane, dimethyloltricyclodecane di(meth)acrylate, trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methyl thio(meth)acrylate, phenyl thio(meth)acrylate, benzyl thio(meth)acrylate, xylylenedithiol di(meth)acrylate, mercaptoethylsulfide di(meth)acrylate, bifunctional urethane (meth)acrylate, and the like.

Specific examples of compounds with allyl groups (allyl compounds) may include allyl glycidyl ether, diallyl phthalate, diallyl terephthalate, diallyl isophthalate, diallyl carbonate, diethylene glycol bisallylcarbonate, methoxypolyethylene glycol allyl ether, polyethylene glycol allyl ether, methoxypolyethylene glycol-polypropylene glycol allyl ether, butoxypolyethylene glycol-polypropylene glycol allyl ether, methacryloyloxypolyethylene glycol-polypropylene glycol allyl ether, phenoxy polyethylene glycol allyl ether, methacryloyloxypolyethylene glycol allyl ether, and the like.

Examples of compounds with vinyl groups, (vinyl compounds) may include α-methylstyrene, α-methylstyrene dimer, styrene, chlorostyrene, methylstyrene, bromostyrene, dibromostyrene, divinylbenzene, 3,9-divinylspirobi(m-dioxane), and the like.

The optical article may include, in any position, one or more layers known as functional layers for optical articles, including a protective layer for improving the durability of the optical article, an anti-reflective layer, a waterrepellent or hydrophilic antifouling layer, an anti-fogging layer, a primer layer for improving the interlaminar adhesive property, and the like.

One embodiment of the optical article is a spectacle lens. In addition, as one embodiment of the optical article, a goggles lens, a visor (cap) part of a sun visor, a shield member of a helmet, and the like may be mentioned. An optical article with anti-glare functions can be obtained by applying the curable composition on a substrate for these optical articles and subjecting the applied composition to curing treatment to form a photochromic layer.

### [Spectacles]

One aspect of the present invention relates to spectacles with the optical article mentioned above. The details of the spectacle lens included in the spectacles are described above. For example, the spectacles provided with such spectacle lenses can exhibit an anti-glare effect, like sunglasses, outdoors by the photochromic compound being irradiated with the sunlight and colored, and in contrast, can recover the permeability when a wearer goes back indoors by the photochromic compound fading the color. Known techniques can be applied to the configuration of the frame and the like for the spectacles.

### [Examples]

Hereinafter, the present invention will be described in more detail with reference to examples. However, the present invention is not limited to the embodiments shown in the examples. The "%" described below represents % by mass.

In the following, a nuclear magnetic resonance apparatus (NMR) was used to identify the molecular structure. As NMR, the proton NMR of ECS-400, manufactured by JEOL Ltd., was used. As measurement solvent, deuterated chloroform was mainly used, and only if it is insoluble in deuterated chloroform, deuterated dimethyl sulfoxide, deuterated acetone, deuterated acetonitrile, deuterated benzene, deuterated methanol, deuterated pyridine, and the like were appropriately used.

Purities were analyzed using high-performance liquid chromatography (HPLC). As an HPLC apparatus, LC-2040C, manufactured by Shimadzu Corporation, was used. YMC-Triart C18 was used as the column, and the measurement temperature was set to 40°C. A mixed solvent of acetonitrile with water containing 0.1% trifluoroacetic acid was used as the mobile phase, and the flow velocity was set to 0.4 mL/min .

For mass spectrometry, an apparatus in which SQD2 was installed as a mass spectrometry unit in ACQUITY UPLC H-Class system (UPLC), manufactured by Nihon Waters K.K., was used. ACQUITY UPLC BEH C18 was used as the column, and the measurement temperature was set to 40°C. A mixed solvent of acetonitrile with water containing formic acid was used as the mobile phase, and the flow velocity was set to 0.61 mL/min with a concentration gradient. The Electrospray ionization (ESI) method was used for ionization.

CHN (carbon, hydrogen, and nitrogen) element analysis was conducted by a combustion method.

### [Example 1]

### <Synthesis of Compound A>

### (First Step)

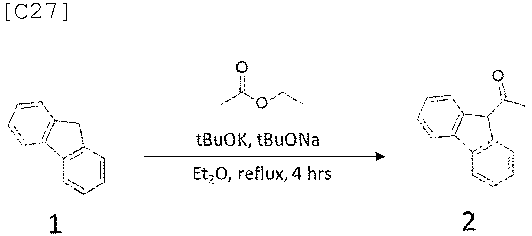

Under an argon atmosphere, potassium tert-butoxide (121 g, 1.08 mol) was added to a diethyl ether solution (1000 mL) of the compound 1 (100 g, 0.60 mol) and refluxed for four hours.

After cooling, 2% aqueous acetic acid solution (1000 mL) was added, and the resulting mixture was stirred for 10 minutes. The mixture was separated into an organic layer and an aqueous layer, the aqueous layer was extracted with ethyl acetate (500 mL × 2). The combined organic layer was washed with water (1000 mL) and then with saturated saline (1000 mL). The washed matter was dried with sodium sulfate, and filtrated and concentrated (150 g). Part (50 g) of the resulting matter was purified by column chromatography (SiO₂: 400 g, chloroform/heptane (volume basis) = 30/70 to 50/50), to yield the compound 2 (38 g). The rest (100 g) was recrystallized in chloroform/heptane (100 mL, 30/70 (volume basis)) to yield the compound 2 (64 g) as a yellow solid.

### (Second Step)

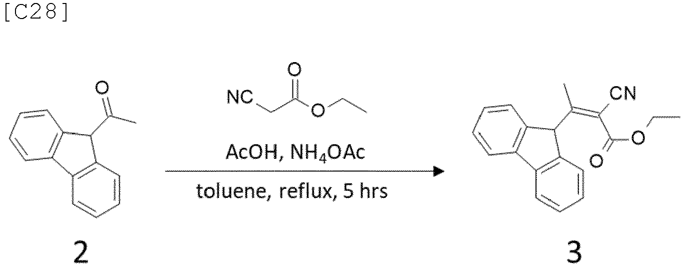

Under an argon atmosphere, a Dean-Stark apparatus was attached to a toluene solution (500 mL) of the compound 2 (100 g, 0.48 mol), ethyl cyanoacetate (54.9 g, 0.49 mol), acetic acid (20 mL, 0.34 mol), ammonium acetate (40.0 g, 0.52 mol) and the solution was refluxed for five hours. After cooling, the resulting mixture was washed sequentially with water (400 mL), saturated carbonated water (200 mL × 2), and saturated saline (200 mL), and the washed matter was dried with sodium sulfate. The dried matter was filtrated and concentrated, and the residue was purified by column chromatography (SiO₂: 1 kg, chloroform/heptane (volume basis) = 50/50 to 75/25) to yield the compound 3 (84.5 g) as a brown oil.

### (Third Step)

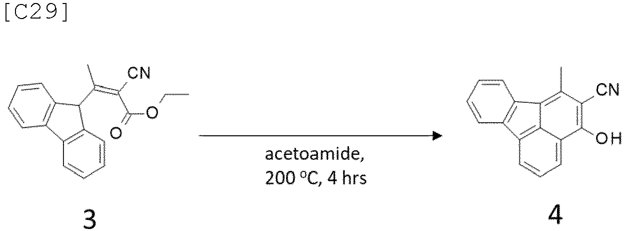

Under an argon atmosphere, the compound 3 (84.5 g, 0.28 mol) and acetamide (90.9 g, 1.53 mol) were stirred at 200°C for 4 hours. A reaction solution cooled to about 90°C was poured into water (1.8 L) at about 70°C and extracted with chloroform (1.8 L). The water layer was extracted with chloroform (0.6 L × 2). The combined organic layer was washed with water (0.6 L × 2), then saturated saline (0.6 L), and dried with sodium sulfate. The dried matter was filtrated and concentrated, and after that, the residue was recrystallized in chloroform/heptane (210 mL) to yield the compound 4 (4.2 g) as a yellow solid.

### (Fourth Step)

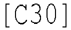

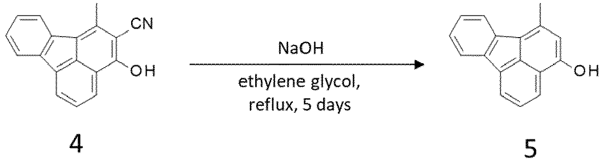

Under an argon atmosphere, sodium hydroxide (7.4 g, 185 mmol) was added to an ethylene glycol solution (80 mL) of the compound 4 (3.7 g, 14.4 mmol), and the mixture was refluxed for 5 days. After cooling, hydrochloric acid (1.0 M, 190 mL) was added, and the resulting mixture was stirred for about 10 minutes. Finally, the precipitated solid was filtrated and dried to yield a mixture of the compound 5 and the compound 4 (compound 4 : compound 5 (mass basis) = 1 : 1, 9.15 g) as a yellow solid.

### (Fifth Step)

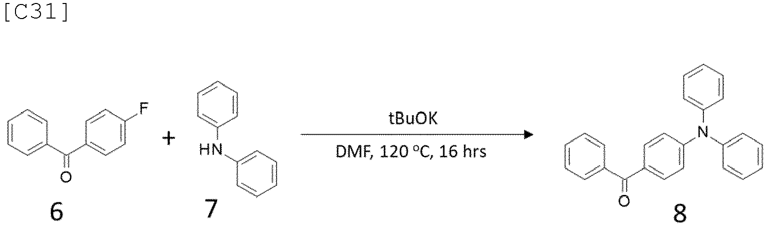

Under an argon atmosphere, the compound 8 (25.0 g, 125 mmol) was added to N,N-dimethylformamide solution (420 mL) of the compound 7 (22.2 g, 131 mmol) and potassium tert-butoxide (14.7 g, 131 mmol), and the mixture was stirred at 120°C for 2 hours.

After cooling, an aqueous saturated ammonium chloride solution (130 mL) was added, and the resulting mixture was extracted with chloroform (200 mL × 3). The organic layer was washed with water (500 mL) and then with saturated saline (100 mL), dried with sodium sulfate, filtrated, and concentrated. The resulting residue was purified by column chromatography (SiO₂: 1 kg, heptane/chloroform (volume basis) = 10/1 to 0/10) to yield the compound 9 (41.1 g) as a yellow oil.

### (Sixth Step)

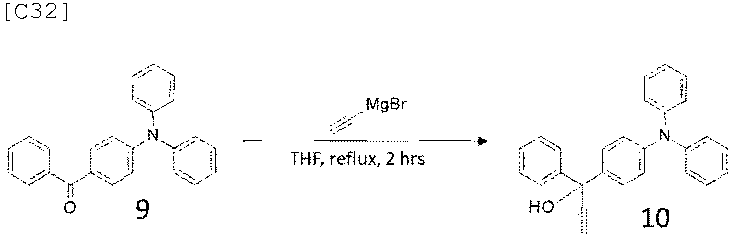

Under an argon atmosphere, a tetrahydrofuran solution (70 mL) of the compound 9 (5.00 g, 14.3 mmol) was cooled with ice, and ethynylmagnesium bromide (0.5 M, 42.9 mL, 21.5 mmol) was added dropwise. The ice bath was removed, and the mixture was stirred at room temperature for 2 hours and refluxed for 2 hours.

After cooling the reaction solution, an aqueous saturated ammonium chloride solution (2 mL) was added, and the resulting mixture was extracted with ethyl acetate (30 mL × 2). The organic layer was washed with water (30 mL) and then saturated saline (30 mL), dried with sodium sulfate, filtrated, and concentrated. The resulting residue was purified by column chromatography (SiO₂: 60 g, heptane/ethyl acetate (volume basis) = 90/10 to 80/20) to yield the compound 10 (4.87 g) as a brown oil.

### (Seventh Step)

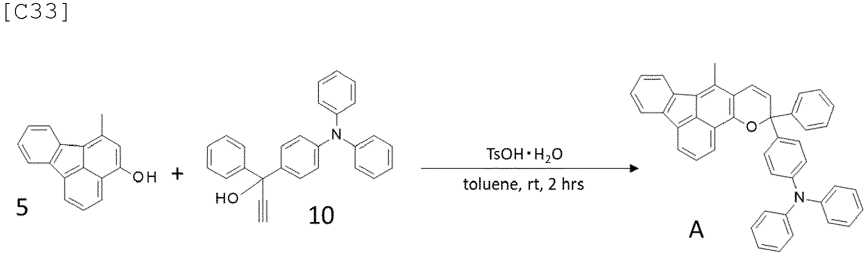

Under an argon atmosphere, p-toluenesulfonic acid monohydrate (0.15 g, 0.80 mmol) was added to a toluene solution (36 mL) of the compound 5 (4.60 g, 3.96 mmol, about 20%) and the compound 10 (2.97 g, 7.92 mmol), and the resulting mixture was stirred overnight at room temperature. An aqueous sodium hydroxide solution (1.0 M, 37 mL) was added, and the mixture was stirred for about 20 minutes. Impurities were removed by filtration, the organic layer was extracted with toluene (30 mL × 2), the combined organic layer was washed with water (20 mL × 2), and concentrated. The resulting residue was purified by column chromatography (SiO₂: 200 g, heptane/chloroform (volume basis) = 70/30 to 60/40) (1.53 g, a brown solid). The resulting solid was suspended in heptane/ethyl acetate (2/1 (volume basis), 90 mL), the suspension was ultrasonicated for about 30 minutes, filtrated, and dried to yield a final product, the compound A (1.39 g), as a light yellow-green solid.

Analysis of the resulting compound A was made by the method described before.

The structure identification was made by a nuclear magnetic resonance apparatus (NMR). The identification result (an NMR chart) is shown in Fig. 1.

Purity analysis by HPLC was found to be 98% in terms of area percentage.

Mass spectrometry revealed that, with respect to the calculated value of 589.241, the measured value was 589.5 (M+, relative strength 100).

A CHN element analysis by a combustion method revealed the following result: with respect to the calculated value C: 89.6%, H: 5.3%, N: 2.4%, the measured value was C: 89.3%, H: 6.6%, N: 2.2%.

The above analysis results comprehensively confirmed that the target compound, compound A, was formed.

### [Examples 2 to 15]

Compounds B to O were obtained by the same operations as above, except that the reaction products listed in Table 2 were respectively used as the compounds 5 and 10 used in the synthesis of the compound A.

The compound was analyzed by the method disclosed earlier. Table 2 shows the analysis result.

### [Comparative Example 1]

Comparative compound 1 was synthesized with reference to Example 1 of WO 2000/15631 (PTL 1). Analysis of the resulting comparative compound 1 was made by the method described before. Table 2 shows the analysis result.

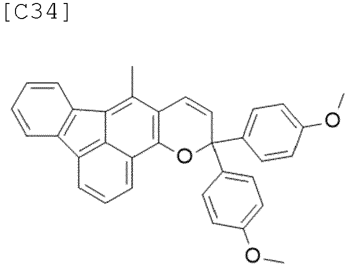

### Comparative Compound 1

### [Evaluation Method]

### <Measurement of Solution Spectrum>

Each of the compounds in Examples and Comparative Examples was dissolved in chloroform that did not contain any stabilizer, and a chloroform solution of each compound was prepared.

A 1-cm square quartz spectroscopic cell containing the prepared solution was covered with a lid and irradiated with a UV ray for 15 seconds using UV-LED (a combination of LIGHTNINGCURE LC-L1V 5 and L14310-120, manufactured by Hamamatsu Photonics K.K., output 70%) as a UV ray source. During irradiation with a UV ray, the solution was stirred by a small-size stirrer.

Within 10 seconds from the end of irradiation with a UV ray, absorbance was measured with an ultraviolet and visible spectrophotometer (UV-1900i, manufactured by Shimadzu Corporation, measurement wavelengths: 700 nm to 350 nm, wavelength 2 nm increments, survey mode). As examples, solution spectra obtained by the measurement on Example 1 and Comparative Example 1 within 10 seconds from the end of irradiation with a UV ray are shown in Fig. 2. The absorbance was measured at room temperature (20°C to 25°C). The concentration of the solution was prepared such that the absorbance at a first absorption wavelength (a peak of absorbance strength observed at the longest wavelength) was 0.95 to 1.05. The quartz spectroscopic cell was left in the spectrophotometer without any light exposure. Thirty minutes later, a spectroscopic measurement was conducted in the same condition, and a first absorption wavelength was found. Table 2 shows the values of the first absorption wavelength and the molar extinction coefficient at that absorption wavelength after the irradiation with a UV ray, and the value of the molar extinction coefficient after leaving for 30 minutes at that absorption wavelength. The quartz spectroscopic cell was removed from the spectrophotometer and irradiated with a UV ray under the same conditions again, and spectroscopic measurements were performed. As a result, it was confirmed that almost the same spectral change was exhibited.

From the measurement result mentioned above, each compound of Examples 1 to 15 exhibited a clear spectral change depending on the presence or absence of irradiation with a UV ray, and it was confirmed that irradiation with a UV ray exhibited photochromic properties in that it undergoes a structural transition to a molecular structure with strong absorption in the visible region by irradiation with a UV ray.

### <Preparation of Photochromic Lens (Spectacle Lens)>

Photochromic lenses (spectacle lenses) were conducted in the following method using each compound in Examples 1 to 15.

### <Preparation of Composition for Photochromic Layer>

In a plastic container, 68% by mass of polyethylene glycol diacrylate (average molecule weight: 508), 12% by mass of trimethylolpropane trimethacrylate (a non-cyclic trifunctional (meth)acrylate), and 20% by mass of neopentyl glycol dimethacrylate, in relation 100% by mass of the total of (meth)acrylates, were mixed to prepare a (meth)acrylate mixture. To this (meth)acrylate mixture, each compound of Examples 1 to 15, a photopolymerization initiator (phenylbis(2,4,6-trimethylbenzoyl)phosphine oxide), an antioxidant [bis(3-tert-butyl-4-hydroxy-5-methylphenyl)propionic acid)]ethylenebis(oxyethylene), and a photostabilizer (bis(1,2,2,6,6-pentamethyl-4-piperidyl) sebacate) were mixed and thoroughly stirred, and a silane coupling agent (γ-methacryloxypropyltrimethoxysilane) was added dropwise under stirring. Then, defoaming was performed with an automatic revolution-type stirring defoaming device.

A composition for a photochromic layer was prepared by the method described above.

### <Formation of Primer Layer>

A plastic lens substrate (trade name EYAS, manufactured by HOYA Corporation, center thickness: 2.5 mm, diameter: 75 mm, spherical lens power: -4.00) was immersed in a 10 mass% aqueous sodium hydroxide solution (liquid temperature: 60°C) for 5 minutes to wash the substrate with alkali, then the substrate was further washed with water and dried. After that, to the convex surface of this plastic lens substrate, aqueous polyurethane resin liquid (polycarbonate polyol-based polyurethane emulsion, viscosity: 100 cPs, solid content concentration: 38% by mass) was applied for 1 minute by a spin coating method at room temperature and a relative humidity of 40% to 60% using a spin coater MS-B150, manufactured by Mikasa Co., Ltd., at a number of revolutions of 1500 rpm, and the resulting coating was dried naturally for 15 minutes to form a primer layer with a thickness of 5.5 µm.

### <Formation of Photochromic Layer>

The composition for the photochromic layer prepared as above was dropped on the primer and coated by a spin coating method using MS-B150, manufactured by Mikasa Co., Ltd., with a program for changing the number of revolutions from 500 rpm to 1500 rpm over one minute by a slop mode and continuing rotating at 1500 rpm for another 5 seconds. After that, the composition for the photochromic layer applied to the primer layer formed on the plastic lens substrate was irradiated with a UV ray (main wavelength: 405 nm) for 40 seconds in a nitrogen atmosphere (oxygen concentration: 500 ppm or less), and this composition was cured to form a photochromic layer. The thickness of the formed photochromic layer was 45 µm.

### [Evaluation of Photochromic Layer]

### <Fading Speed>

A fading speed was evaluated by the following method.

The transmittance (measurement wavelength: 550 nm) of each photochromic lens prepared as above before the irradiation with light (uncolored state) was measured by a spectrophotometer, manufactured by Otsuka Electronics Co., Ltd. The transmittance measured in this manner is referred to as an "initial transmittance".

Each photochromic lens was irradiated with light emitted from a xenon lamp as a light source and passed through an aeromass filter for 15 minutes to color each photochromic compound in the photochromic layer. The irradiation with light was performed so that irradiances and tolerances of irradiances specified in JIS T 7333:2005 were values shown in Table 1. The transmittance at this coloration was measured, similarly to an initial transmittance. The transmittance measured in this manner is referred to as a "transmittance at coloration".

After that, the period from the time at which irradiation with light was stopped to the time at which the transmittance reached [(initial transmittance - transmittance at coloration)/2] was measured. This period is referred to as a "half-value period". The shorter the half-value period, the faster the fading rate. Determined half-value periods are listed in Table 2.

From the half-value periods listed in Table 2, it was confirmed that the spectacle lens containing each photochromic compound in Examples 1 to 15 functioned as a photochromic lens, as with spectacle lenses commercially available as common photochromic lenses.

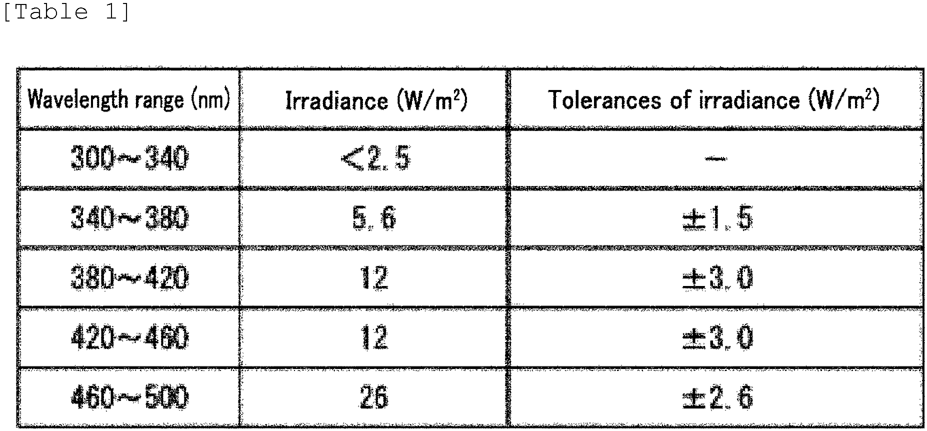

Table 2 (Tables 2-1 to 2-4) shows the above results.

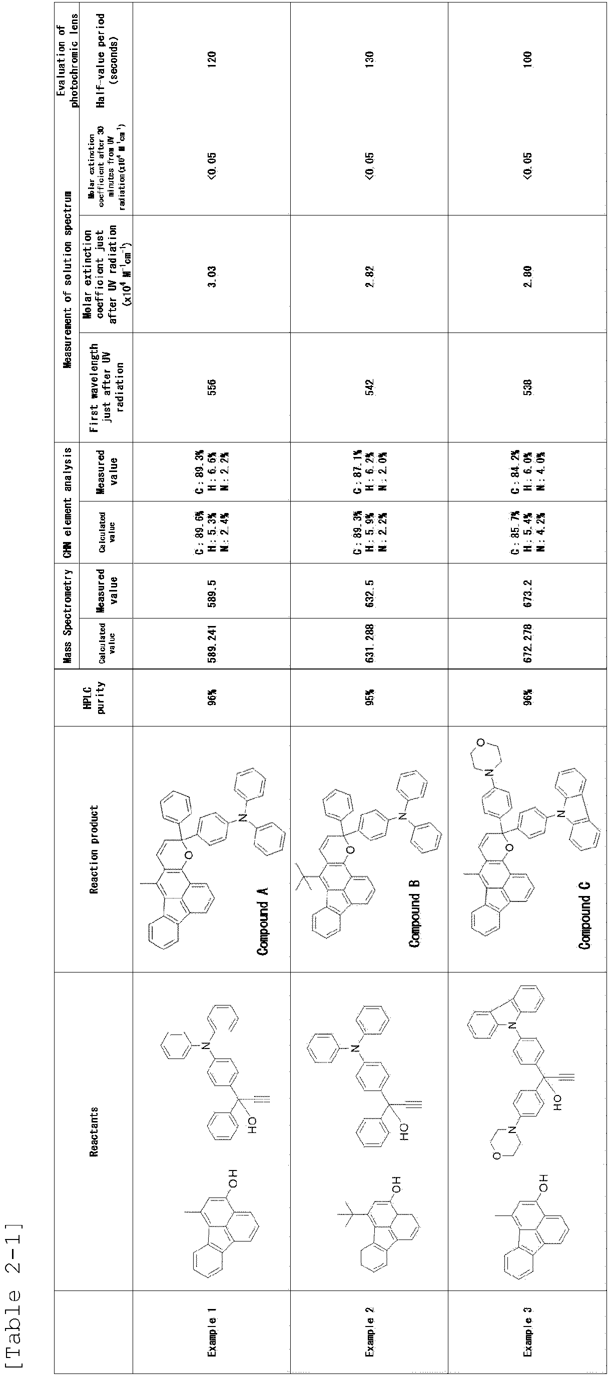

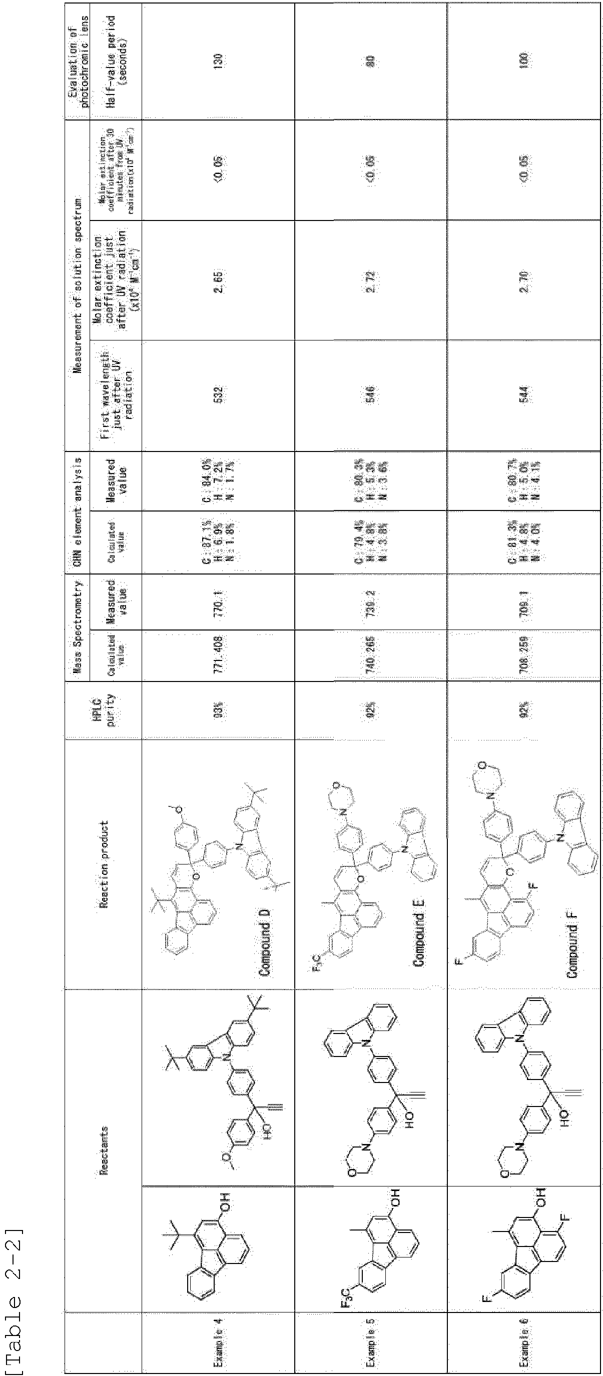

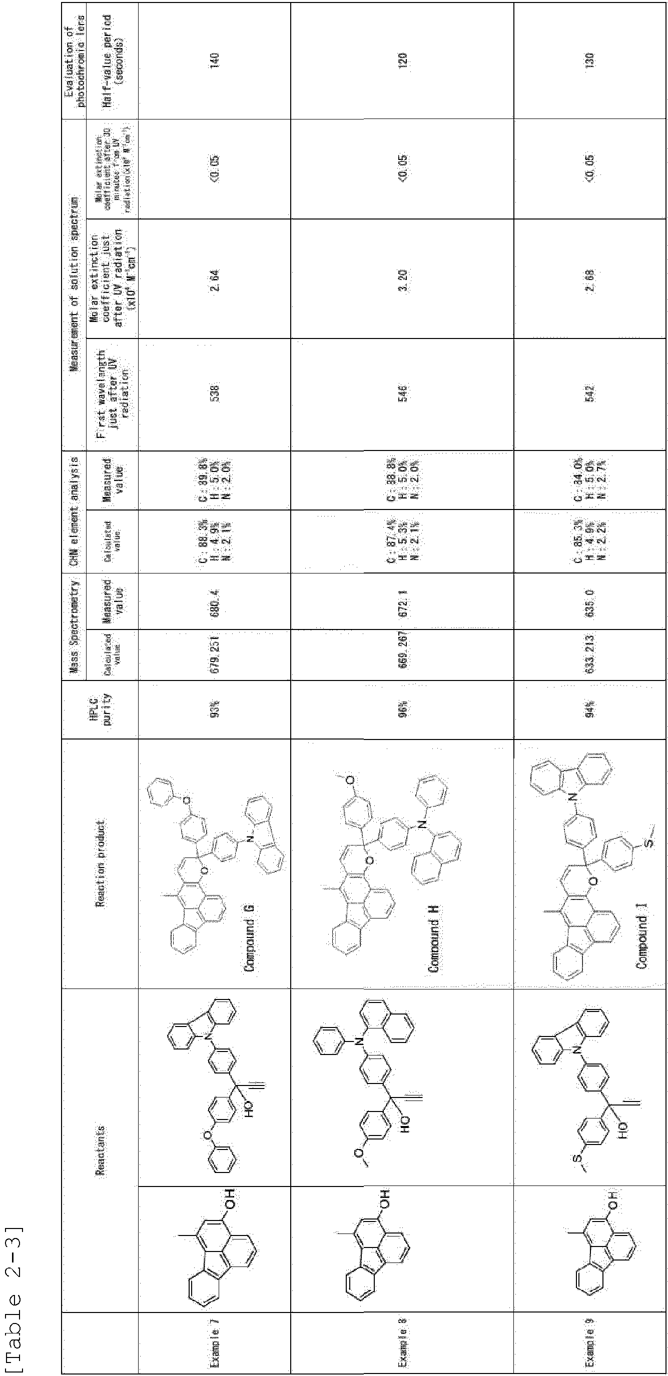

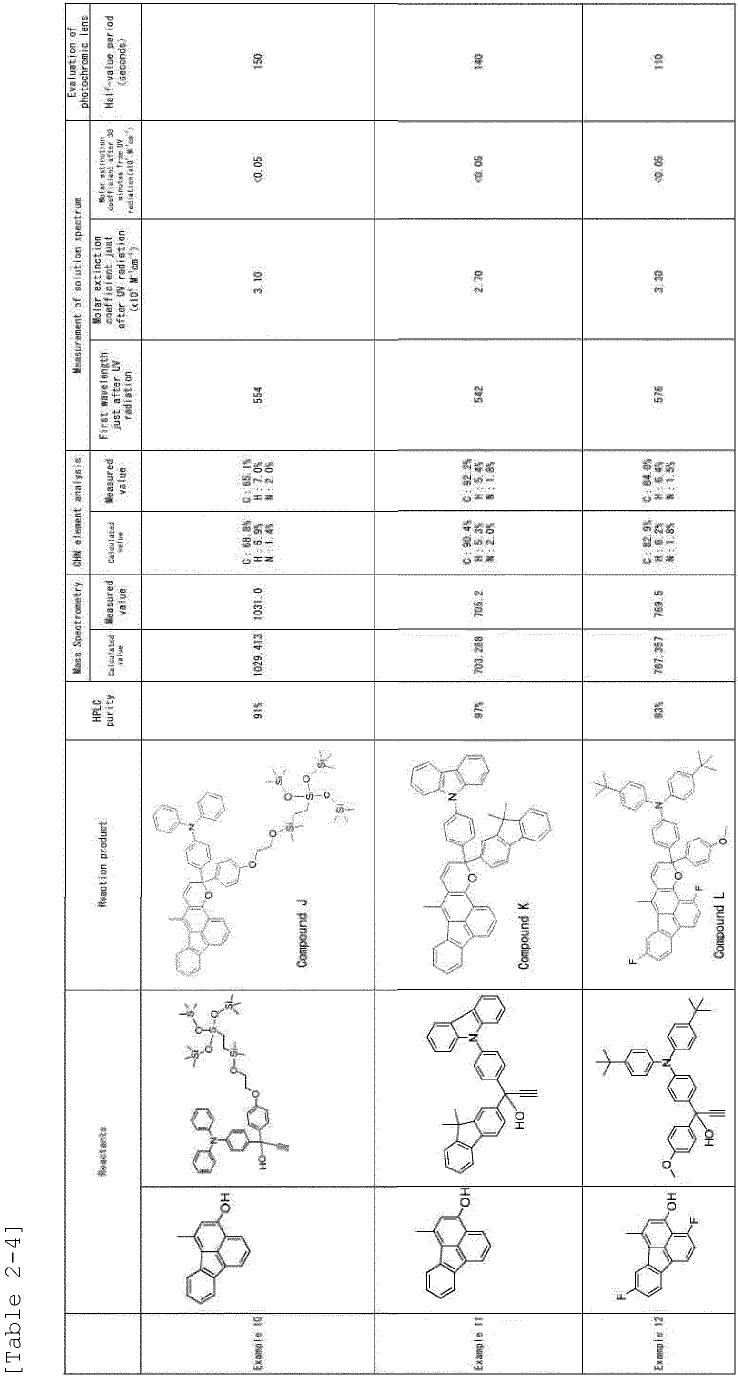

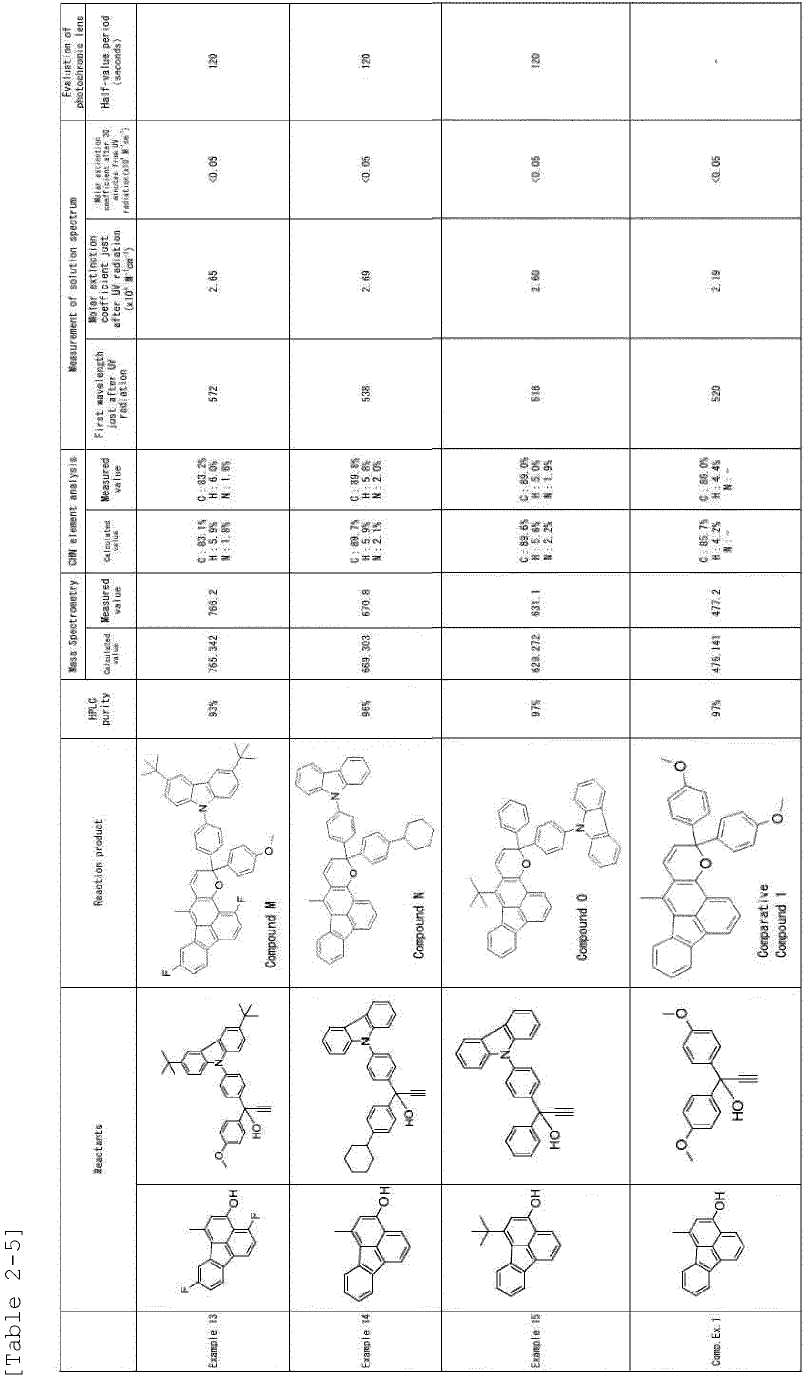

Finally, the above aspects will be summarized.

According to one embodiment, an optical article including one or more photochromic compounds selected from the group consisting of a compound represented by general formula 1 and a compound represented by general formula 2 is provided.

In addition, according to one aspect, a photochromic compound represented by general formula 1 and a photochromic compound represented by general formula 2 are provided.

The photochromic compound can show high absorbing strength.

In one embodiment, the optical article may at least contain one or more compounds represented by the general formula 1, and, in the general formula 1, one of B¹ and B² may be a monovalent group represented by the general formula 3 and the other may be a monovalent group represented by general formula 4.

In one embodiment, the optical article may at least contain one or more compounds represented by general formula 1, and, in the general formula 1, Ar¹ in the monovalent group represented by the general formula 3 may represent an aryl group.

In one embodiment, the optical article may at least contain one or more compounds represented by the general formula 1, and, in the general formula 1, R¹⁸ in the monovalent group represented by the general formula 3 may represent an aryl group.

In one embodiment, the optical article may at least contain one or more compounds represented by general formula 2, and, in the general formula 2, one of B³ and B⁴ may be a monovalent group represented by the general formula 3 and the other may be a monovalent group represented by general formula 4.

In one embodiment, the optical article may at least contain one or more compounds represented by the general formula 2, and, in the general formula 2, Ar¹ in the monovalent group represented by the general formula 3 may represent an aryl group.

In one embodiment, the optical article may at least contain one or more compounds represented by general formula 2, and, in the general formula 2, R¹⁸ in the monovalent group represented by the general formula 3 may represent an aryl group.

In one embodiment, the optical article may include a substrate and a photochromic layer containing the photochromic compound.

In one aspect, the optical article may be a spectacle lens.

In one aspect, the optical article may be a goggles lens.

In one aspect, the optical article may be a visor part of a sun visor.

In one aspect, the optical article may be a shield member of a helmet.

According to one aspect, spectacles including the spectacle lens are provided.

Two or more of the various aspects and embodiments described in this specification may be combined in any combination.

The embodiments disclosed herein are only examples in all respects and should not be considered as restrictive. The scope of the present invention is not limited to the above description, but is defined by the scope of claims, and is intended to encompass equivalents to the scope of claims and all modifications within the scope of the claims.

### [Industrial Applicability]

One aspect of the present invention is beneficial in the technical fields of spectacles, goggles, sun visors, helmets, and the like.

## Claims

1. An optical article comprising one or more photochromic compounds selected from the group consisting of a compound represented by general formula 1 below and a compound represented by general formula 2 below. 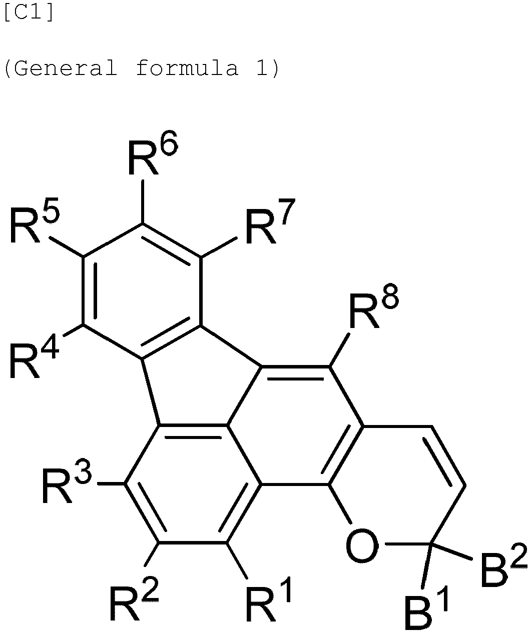 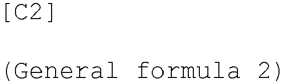 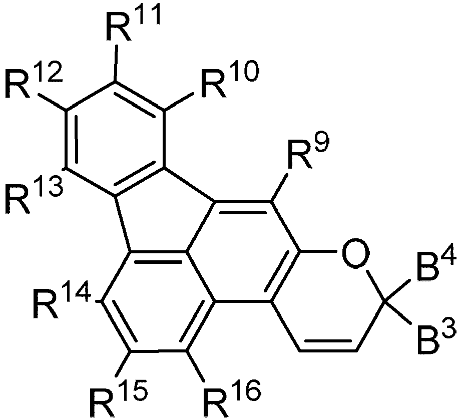
(in the general formula 1, R¹ to R⁸, and B¹ and B² each independently represent a hydrogen atom or a substituent, and at least either of B¹ and B² represents a monovalent group represented by general formula 3 below; and
in the general formula 2, R⁹ to R¹⁶, and B³ and B⁴ each independently represent a hydrogen atom or a substituent, and at least either of B³ and B⁴ represents a monovalent group represented by general formula 3 below; 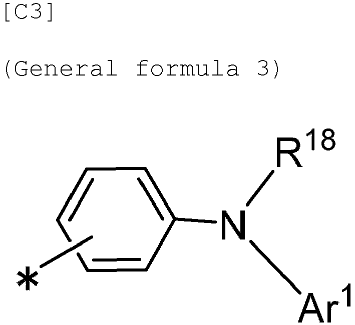
in the general formula 3, Ar¹ represents an aryl group or a heteroaryl group, R¹⁸ represents a hydrogen atom or a substituent, Ar¹ and R¹⁸ may be coupled to form a ring structure via a single bond or a divalent linking group, and * represents a bonding position to an adjacent carbon atom).

2. The optical article according to claim 1, at least comprising one or more compounds represented by the general formula 1, wherein
in the general formula 1, one of B¹ and B² is a monovalent group represented by the general formula 3, and the other is a monovalent group represented by general formula 4 below. 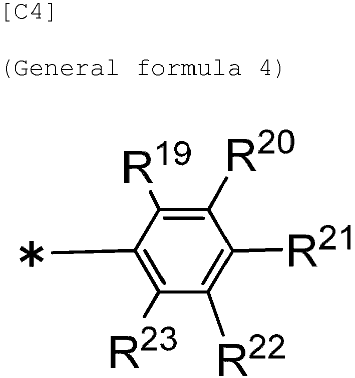
(in the general formula 4, R¹⁹ to R²³ each independently represent a hydrogen atom or a substituent.)

3. The optical article according to claim 1 or 2, at least comprising one or more compounds represented by the general formula 1, wherein
in the general formula 1, Ar¹ in the monovalent group represented by the general formula 3 represents an aryl group.

4. The optical article according to any one of claims 1 to 3, at least comprising one or more compounds represented by the general formula 1, wherein
in the general formula 1, R¹⁸ in the monovalent group represented by the general formula 3 represents an aryl group.

5. The optical article according to claim 1, at least comprising one or more compounds represented by the general formula 2, wherein
in the general formula 2, one of B³ and B⁴ is a monovalent group represented by the general formula 3, and the other is a monovalent group represented by general formula 4 below. 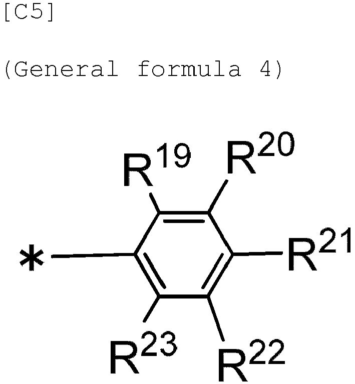
(in the general formula 4, R¹⁹ to R²³ each independently represent a hydrogen atom or a substituent.)

6. The optical article according to claim 1 or 5, at least comprising one or more compounds represented by the general formula 2, wherein
in the general formula 2, Ar¹ in the monovalent group represented by the general formula 3 represents an aryl group.

7. The optical article according to claim 1, 5, or 6, at least comprising one or more compounds represented by the general formula 2, wherein
in the general formula 2, R¹⁸ in the monovalent group represented by the general formula 3 represents an aryl group.

8. The optical article according to any one of claims 1 to 7, comprising a substrate and a photochromic layer containing the photochromic compound.

9. The optical article according to any one of claims 1 to 8, wherein the optical article is a spectacle lens.

10. The optical article according to any one of claims 1 to 8, wherein the optical article is a goggle lens.

11. The optical article according to any one of claims 1 to 8, wherein the optical article is a visor portion of a sun visor.

12. The optical article according to any one of claims 1 to 8, wherein the optical article is a shield member of a helmet.

13. Spectacles comprising the spectacle lens according to claim 9.

14. A photochromic compound represented by general formula 1 below. 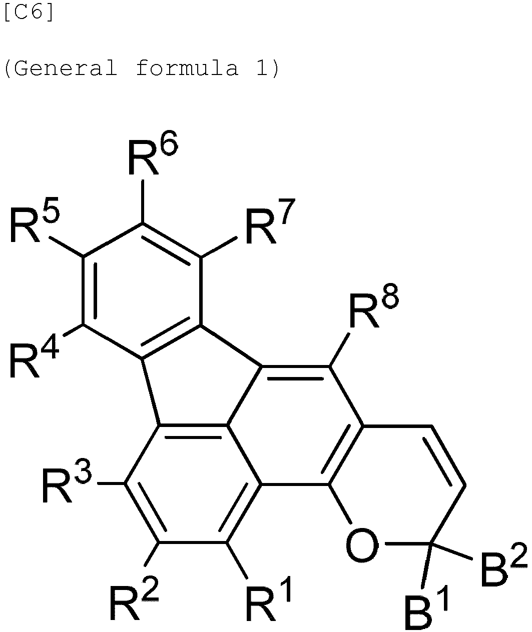
(in the general formula 1, R¹ to R⁸, and B¹ and B² each independently represent a hydrogen atom or a substituent, and at least either of B¹ and B² represents a monovalent group represented by general formula 3 below; 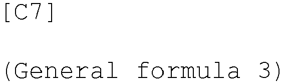 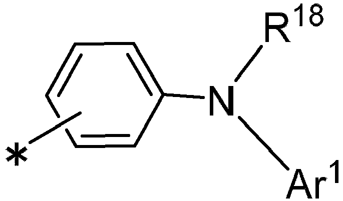
in the general formula 3, Ar¹ represents an aryl group or a heteroaryl group, R¹⁸ represents a hydrogen atom or a substituent, Ar¹ and R¹⁸ may be coupled to form a ring structure via a single bond or a divalent linking group, and * represents a bonding position to an adjacent carbon atom).

15. The photochromic compounds according to claim 14, wherein, in the general formula 1, one of B¹ and B² is a monovalent group represented by the general formula 3, and the other is a monovalent group represented by general formula 4 below. 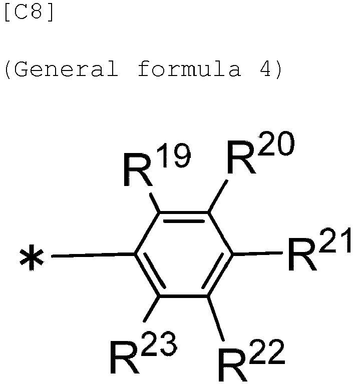 (in the general formula 4, R¹⁹ to R²³ each independently represent a hydrogen atom or a substituent.)

16. The photochromic compound according to claim 14 or 15, wherein, in the general formula 1, Ar¹ in the monovalent group represented by the general formula 3 represents an aryl group.

17. The photochromic compound according to any one of claims 14 to 16, wherein, in the general formula 1, R¹⁸ in the monovalent group represented by the general formula 3 represents an aryl group.

18. A photochromic compound represented by general formula 2 below. 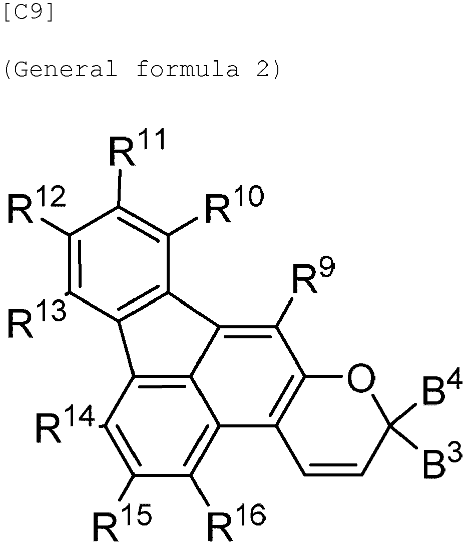
(in the general formula 2, R⁹ to R¹⁶, and B³ and B⁴ each independently represent a hydrogen atom or a substituent, and at least either of B³ and B⁴ represents a monovalent group represented by general formula 3 below; 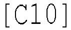 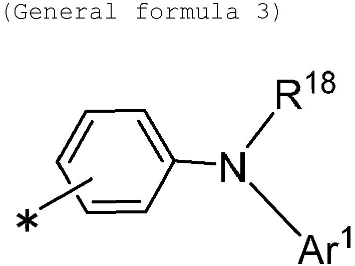
in the general formula 3, Ar¹ represents an aryl group or a heteroaryl group, R¹⁸ represents a hydrogen atom or a substituent, Ar¹ and R¹⁸ may be coupled to form a ring structure via a single bond or a divalent linking group, and * represents a bonding position to an adjacent carbon atom).

19. The photochromic compounds according to claim 18, wherein, in the general formula 2, one of B³ and B⁴ is a monovalent group represented by the general formula 3, and the other is a monovalent group represented by general formula 4 below. 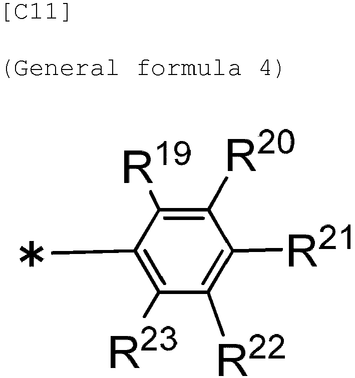 (in the general formula 4, R¹⁹ to R²³ each independently represent a hydrogen atom or a substituent.)

20. The photochromic compound according to claim 18 or 19, wherein, in the general formula 2, Ar¹ in the monovalent group represented by the general formula 3 represents an aryl group.

21. The photochromic compound according to any one of claims 18 to 20, wherein, in the general formula 2, R¹⁸ in the monovalent group represented by the general formula 3 represents an aryl group.
